# EUROPEAN PATENT APPLICATION

(11) **EP 2 163 639 A1**
(43) Date of publication of application: **17.03.2010**
(21) Application number: 08163692.0
(22) Date of filing: 04.09.2008
(51) Int. Cl.: C12N 15/82, C07K 14/415

(54) **New tomato ethylene response factors and uses thereof**

(71) Applicant: Institute National Polytechnique de Toulouse, 31000 Toulouse (FR)
(72) Inventor: Bouzayen, Mondher, 31320 Auzeville-Tolosane (FR); Latche, Alain, 31100 Toulouse (FR); Pech, Jean-Claude, 31400 Toulouse (FR); Pirrello, Julien, 31400 Toulouse (FR); Regad, Farid, 31200 Toulouse (FR)
(74) Representative: Tetaz, Franck Claude Edouard

(57) **Abstract**

The present invention concerns a tomato transcription factor of the Ethylene Responsive Factor family (ERF), having an amino acid sequence selected among the group consisting in :
- a protein having an amino acid sequence as shown in one of SEQ ID NO 1 to NO 28,
- a variant thereof,
- a functional fragment thereof and
- a functional homologous sequence thereof.

## Description

### INTRODUCTION

The plant hormone ethylene is involved in the control of myriad plant developmental processes and is also known to play an active role in plant responses to biotic and abiotic stresses. It is well established that this plant hormone exerts its effects by modulating the expression of a large number of genes through the activation of specific members of the Ethylene Response Factor (ERF) gene family. ERFs are plant specific transcriptional regulators encoded by one of the largest gene family known in plant kingdom. Because ERFs are down-stream mediators of ethylene action, they are the main actors for the amplification and diversification of plant responses to ethylene.

The making of a fruit is a developmental process unique to plants, involving a complex network of interacting genes and signalling pathways. In the case of fleshy fruit, this process involves three main stages : (a) fruit set, (b) fruit development, and (c) fruit ripening. This latter stage is crucial for fruit quality and most of the sensory and health promoting compounds accumulate during the ripening step.

Ripening is a genetically programmed process orchestrated by complex interplay between endogenous hormones and environmental cues. The regulated changes at the level of gene expression are the first steps leading to the metabolic changes associated with fruit ripening. In climacteric fruit type like tomato, the phytohormone ethylene is a key regulator of this process.

However, while molecular and genetic evidences are becoming available showing that ethylene triggers and co-ordinates fruit ripening, the developmental cues required to signal a readiness to ripen remain unknown and the mechanism by which the fruit developmental program switches into a ripening process is yet to be elucidated.

Transcription factors from the ERF type are thought to be, at least partly, responsible of the complex network of metabolic activations associated with the ripening process, nevertheless, the mechanism by which they select ripening-specific genes remains largely unknown.

### PRIOR ART

At first, Ethylene Responsive Factors were isolated as GCC box binding proteins from tobacco (Ohme-Takagi and Shinshi, 1995). Later, ERFs have been identified in numerous plants including *Arabidopsis Thaliana* and *Solanum lycopersicum* (tomato).

A highly conserved DNA binding domain, known as the "AP2/ERF domain", consisting of 58 to 59 amino acids, is the unique structural feature of common to all factors belonging to this protein family. This ERF domain binds to DNA as a monomer, with high affinity.

ERFs can act as either transcriptional activators or repressors for GCC box-dependant gene expression (Fujimoto et al., Plant Cell, 2000).

ERFs have been shown to be involved in normal and abnormal plant processes such as plant defense, osmotic stress tolerance, and seed germination.

In tomato, new members of the ERF family were identified in 2003 and their respective role and expression pattern were studied (Tournier et al., FEBS Letters, 2003). Four distinct classes of ERF were described, based on functionalities and structures of each subtype.

Later, the physiological function of one of the identified ERF was extensively studied. In particular, overexpression of said ERF gene in transgenic tomato line results in premature seed germination and increased ethylene sensitivity. Interestingly, the *mannanase2* encoding gene is upregulated in seeds (Pirrello et al., 2006).

This preliminary data indicate that each ERF controls a specific subset of ethylene-regulated genes. Therefore, identifying a set of ripening-associated genes that are under the regulation of a given ERF will open new prospects towards the targeted control of genes involved in specific metabolic pathways, and more particularly in ripening control.

### DESCRIPTION OF THE INVENTION

The present invention is related to new ERF proteins, identified in tomato, classified according to their structural characteristics, expression pattern and physiological functions.

The invention is also related to the modulation of the expression of genes encoding said ERFs, these activation or repression being used to obtain new interesting phenotypes of tomato plants.

Moreover, mapping of genes encoding ERF proteins, and identification of genetic markers QTL linked to these genes, are claimed here.

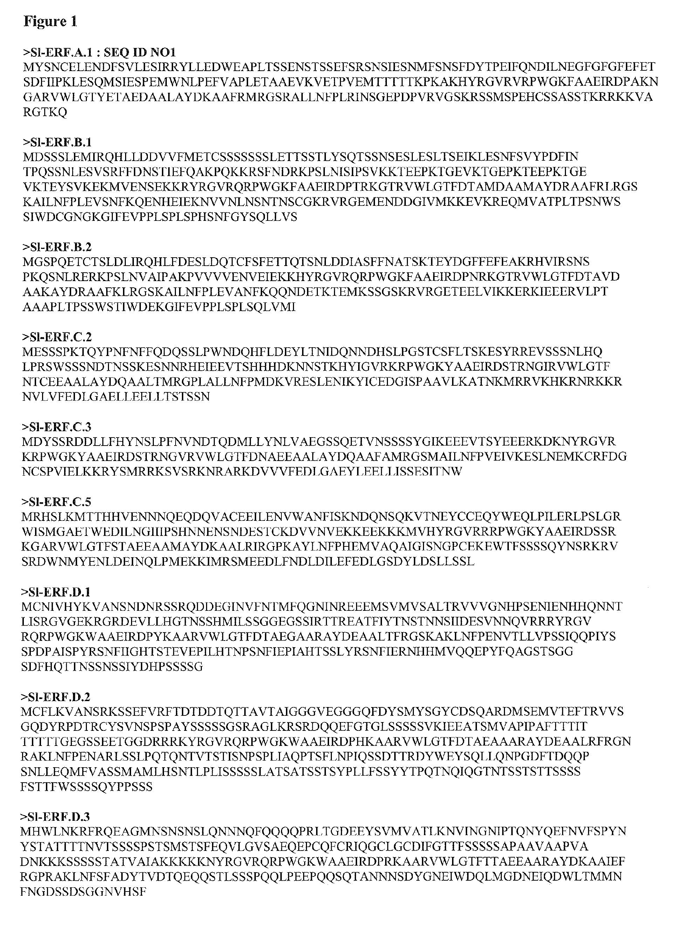

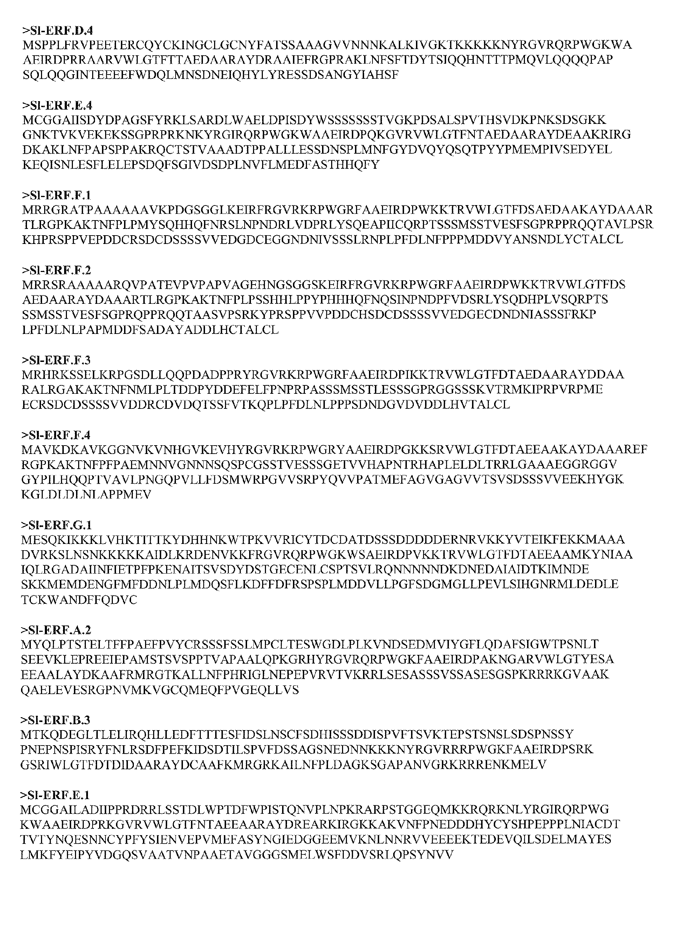

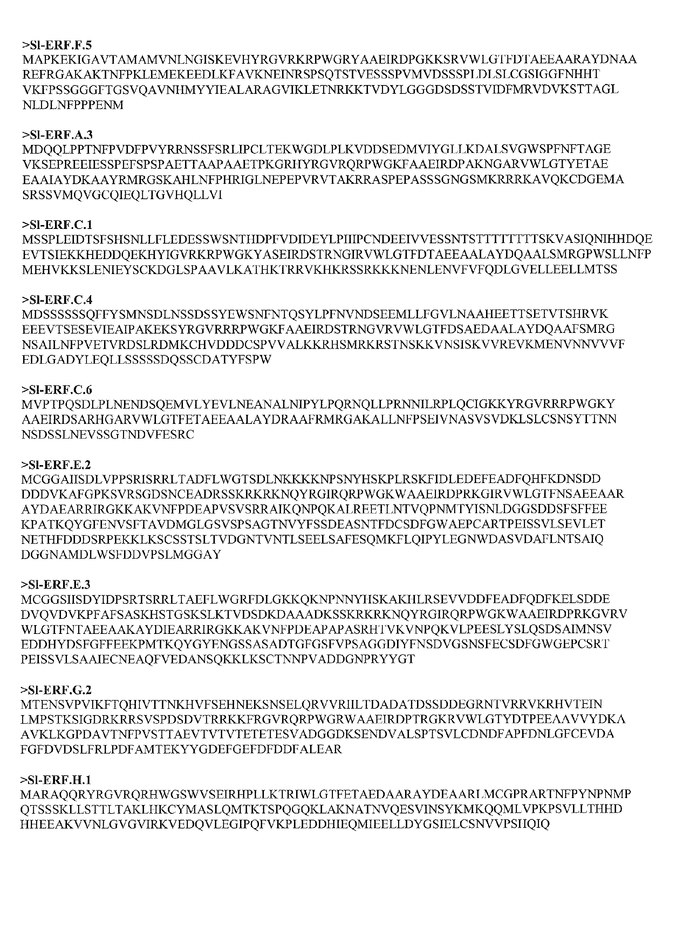

Screening of various cDNA libraries from different plant tissues and organs allowed the isolation of complete cDNA clones for 28 tomato ERFs. Complete amino acid sequences of the ERF proteins are provided in Figure 1 and in the Sequence Listing; corresponding polynucleotide sequences of the cDNA are provided in the sequence listing.

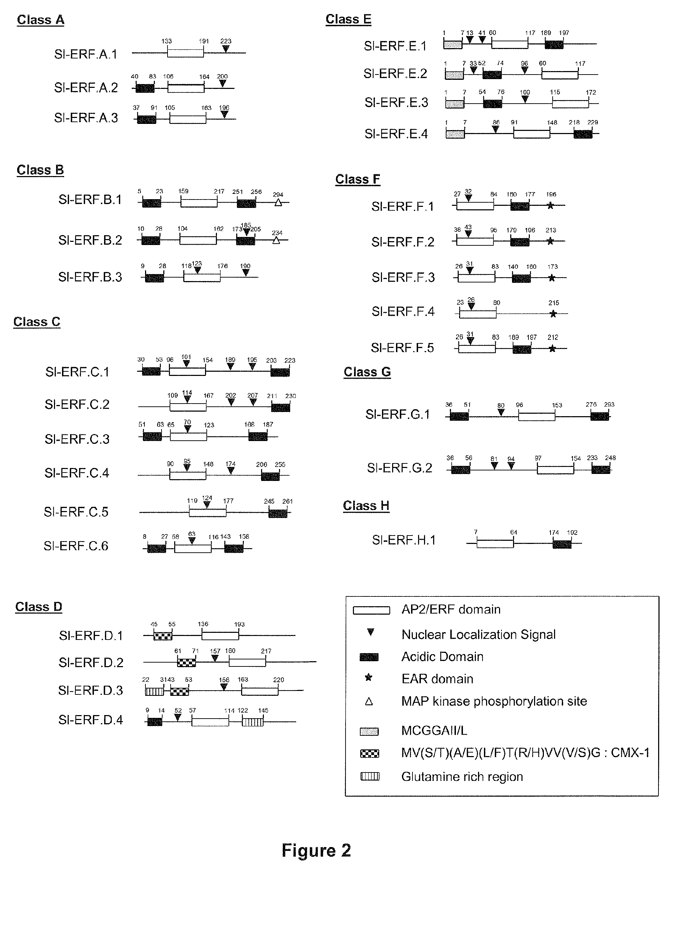

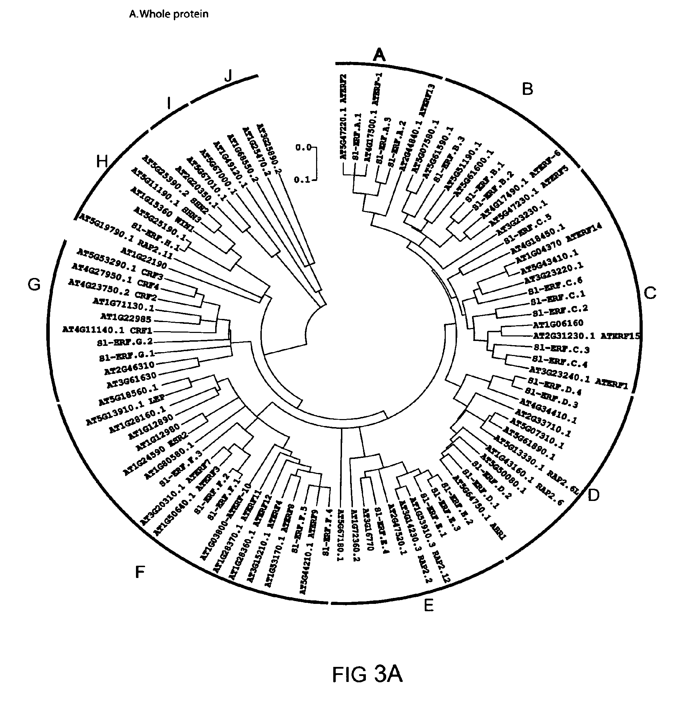

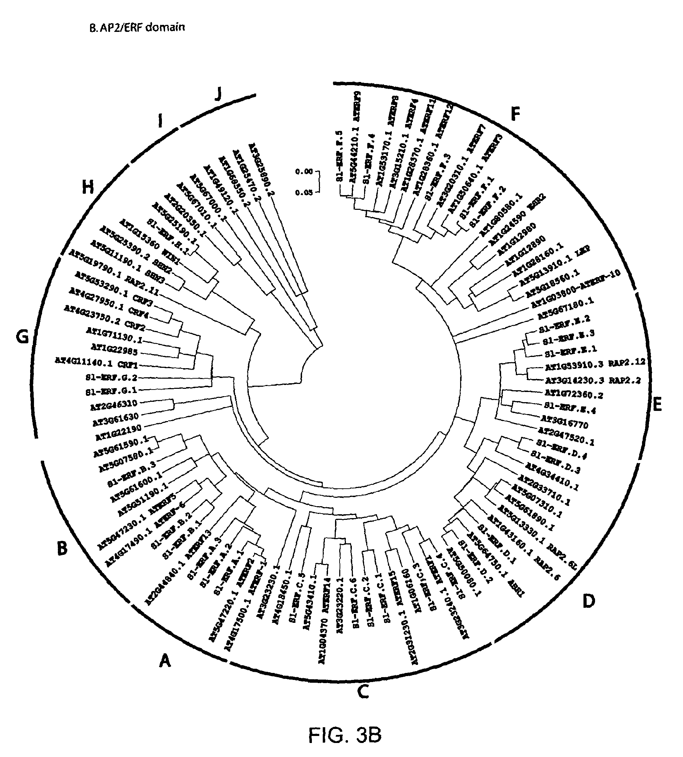

In silico analysis allowed to classify the encoded proteins based on their structural features and by comparison with the corresponding Arabidopsis (*Arabidopsis thaliana*) and rice (*Oryza sativa L. subsp. japonica*) orthologs. Phylogenetic analyses defined eight ERF sub-classes in the tomato; each of them display distinct specific features (Figures 2 and 3).

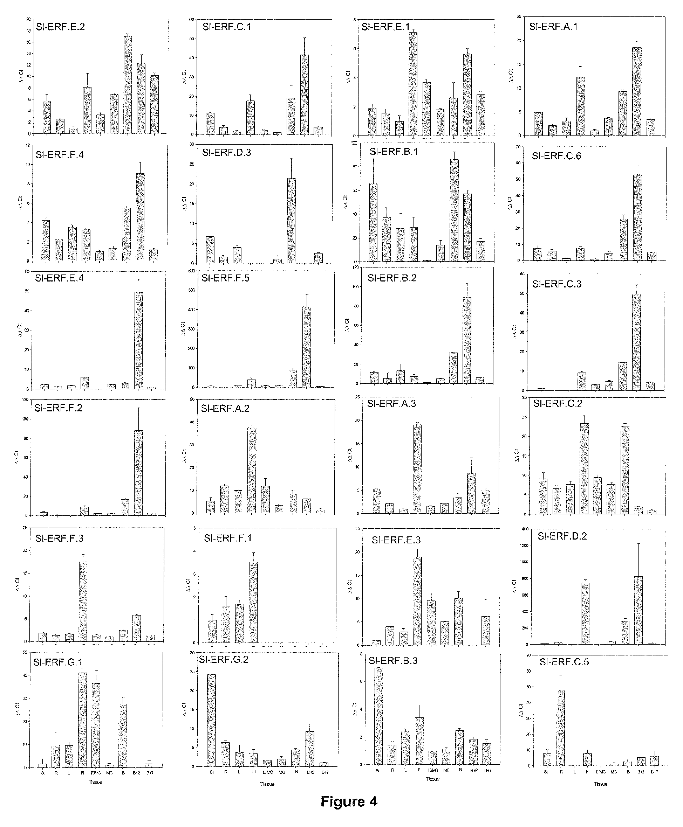

The spatio-temporal pattern of expression of some members of the tomato ERF gene family was established which indicated that some ERFs are preferentially expressed in the fruit, other ERFs in flower organ and a few in vegetative tissues, while the remaining ERFs displays a constitutive pattern of expression (Figure 4).

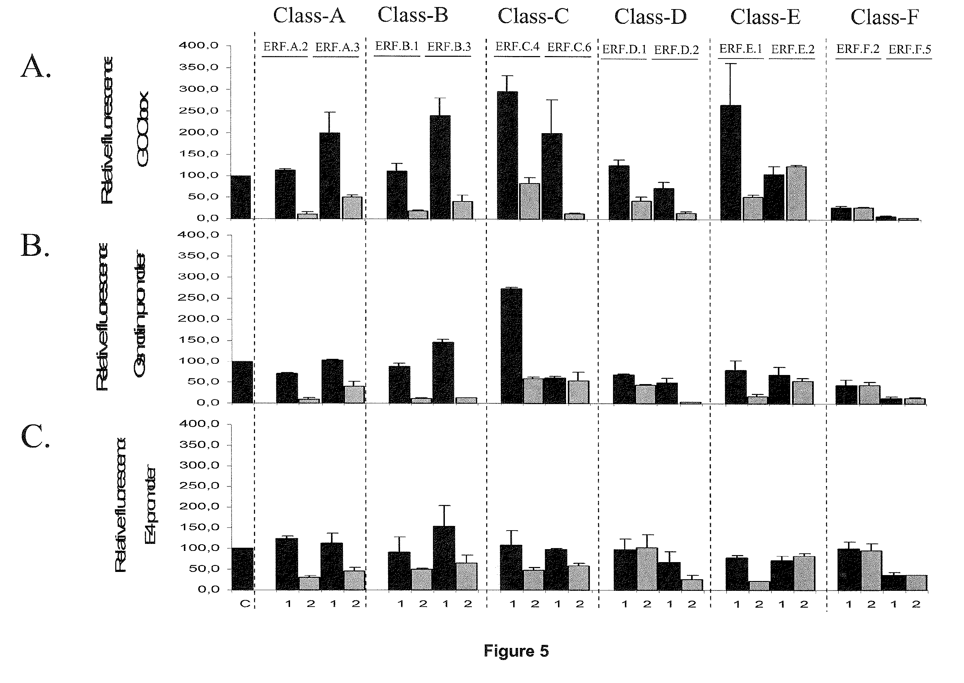

The ability of each ERF to regulate *in vivo* the activity of ethylene-responsive promoters, containing or not a GCC box, was assayed. Depending on the sub-class to which they belong, ERF proteins can act either as positive or negative regulators of GCC-containing promoters. Moreover, some ERFs can also modulate the transcriptional activity of native ethylene-responsive promoters lacking the canonical GCC box (Figure 5).

All together, the structural diversity, the preferential binding to target promoters as well as the differential patterns of expression, provide new insights on how ERF proteins mediate ethylene responses in highly selective and specific manner.

Among the numerous applications that arise from the isolation and functional characterization of tomato ERFs, the following are of first importance:
- Obtaining ERF-drived markers for plant breeding via marker-assisted classical genetics approaches;
- Selection of naturally occurring or chemically induced mutants having commercially relevant phenotypes including fruit traits, tolerance to biotic and abiotic stresses;
- Up-regulating and down-regulating the expression of ERFs via transgenesis in homologous or heterologous hosts to generate novel and advantageous plant properties.

### DETAILLED DESCRIPTION OF THE INVENTION

The present invention is related to new transcription factors from tomato, belonging to the Ethylene Responsive Factor family (ERF), showing an amino acid sequence chosen among SEQ ID NO 1 to NO 28, a variant thereof, a functional fragment thereof and a functional homologous sequence thereof.

The term of « transcription factor » designates a protein that binds to promoters of genes, using DNA binding domains, and controls the transcription of the gene, i.e. the level of expression of the gene.

The term of "Ethylene Responsive Factor" or "ERF" designates a transcription factor having as DNA binding domain, a domain called "AP2/ERF" consisting of 58 to 59 amino acids, highly conserved among all the factors of the family.

The term "functional fragment" means according to the invention that the sequence of the polypeptide may include less amino-acid than shown in SEQ ID N°1 to N°28 but still enough amino acids to confer the Ethylene Responsive Factor activity. It is well known in the art that a polypeptide can be modified by substitution, insertion, deletion and/or addition of one or more amino acids while retaining its DNA binding activity. For example, substitutions of one amino-acid at a given position by a chemically equivalent amino-acid that do not affect the functional properties of a protein are common. For the purposes of the present invention, substitutions are defined as exchanges within one of the following groups:
■ Small aliphatic, non-polar or slightly polar residues : Ala, Ser, Thr, Pro, Gly
■ Polar, negatively charged residues and their amides : Asp, Asn, Glu, Gln
■ Polar, positively charged residues : His, Arg, Lys
■ Large aliphatic, non-polar residues : Met, Leu, Ile, Val, Cys
■ Large aromatic residues : Phe, Tyr, Trp.

Thus, changes which result in substitution of one negatively charged residue for another (such as glutamic acid for aspartic acid) or one positively charged residue for another (such as lysine for arginine) can be expected to produce a functionally equivalent product.

The positions where the amino acids are modified and the number of amino acids subject to modification in the amino acid sequence are not particularly limited. The man skilled in the art is able to recognize the modifications that can be introduced without affecting the activity of the protein. For example, modifications in the N- or C-terminal portion of a protein would not be expected to alter the activity of a protein.

The term "functional homologous" and "variant" refers to polypeptides submitted to modifications such as defined above while still retaining the original enzymatic activity.

In a specific embodiment of the invention, the polypeptide of the present invention have at least 70% identity with the sequences shown as SEQ ID N°1 to SEQ ID N°28, preferentially at least 80% identity and more preferentially at least 90% identity.

Methods for determination of the percentage of identity between two protein sequences are known from the man skilled in the art. For example, it can be made after alignment of the sequences by using the software CLUSTALW available on the website http://www.ebi.ac.uk/clustalw/ with the default parameters indicated on the website. From the alignment, calculation of the percentage of identity can be made easily by recording the number of identical residues at the same position compared to the total number of residues. Alternatively, automatic calculation can be made by using for example the BLAST programs available on the website http://www.ncbí.nlm.nih.gov/BLAST/ with the default parameters indicated on the website.

In a particular embodiment of the invention, ERFS are selected among the proteins having an amino acid sequence as shown in SEQ ID NO 1 to 20, and more preferentially in SEQ ID NO 1 to 16.

In another embodiment of the invention, ERFS are selected among DNA fragments having a nucleotidic sequence as shown in one of SEQ ID N0 29 to NO 56 or a functional homologous sequence thereof.

According to the invention, the tomato ERFs present specific features, and in particular at least one of the following structural features:
- a nuclear localization signal; almost all ERFs according to the invention show this signal, except ERF.D1 and H1. Presence of a nuclear localization signal strongly supports that the encoded ERF proteins are targeted to the cell nucleus, and therefore act, positively or negatively, on transcription regulation;
- an acidic domain; almost all ERF according to the invention show an acidic domain, except ERF.A1, D1, D2, D3 and F4. Presence of an acidic domain suggests that the corresponding ERF proteins display transcriptional activation properties;
- an EAR domain; presence of a putative EAR motif strongly supports that the corresponding ERF proteins display some transcriptional repression properties. This characteristic is specific to ERF belonging to the class F : F1 to F5;
- a putative MAP kinase phosphorylation site ; presence of a MAP kinase phosphorylation site suggests that the corresponding ERF proteins bind efficiently on the GCC box present in DNA;
- a sequence "MCGGAII/L"; this structural characteristic is found in all factors of class E. Its function is still unknown;
- a sequence CMX-1; this structural characteristic is found in all factors of class D but D4. Its function is still unknown;
- a glutamine rich region; this structural characteristic is found in factors ERF.D3 and D4. Its putative function is an activation of the transcription.

Some ERFs according to the invention present a combination of two or more of the features presented above.

The spatio-temporal pattern of expression of twenty-four members of the tomato ERF gene family was established, which indicated that some ERFs are preferentially expressed in the fruit, others in flower organ or in both flower and fruit, a few are expressed mainly in vegetative tissues, while the remaining ERFs display a constitutive pattern of expression.

The knowledge of the expression pattern of these ERFs is of primary importance to anticipate the physiological effects of a modulation of expression of these factors.

Each of the tomato ERFs according to the invention can act positively or negatively on the transcription of responsive genes. Indeed, the ERF proteins are known to modulate transcription of ethylene-responsive genes via binding to the so-called GCC box, a *cis-*elements found in the promoter region of ethylene-regulated genes.

A "responsive gene" is defined as a gene having a promoter on which the ERF can bind, and the expression level of said gene is modulated positively or negatively by the binding of the ERF.

Each ERF has the functional ability to regulate *in vivo* the activity of ethylene-responsive promoters. This ability was assayed by using a dedicated transient expression system.

The data presented in example 2 (below) indicate that depending on the sub-class to which they belong, ERF proteins can act either as positive or negative regulators of GCC-containing promoters.

Surprisingly, inventors show also that some ERFs can also modulate the transcriptional activity of native ethylene-responsive promoters lacking the canonical GCC box (Figure 5 and table 2).

In a specific embodiment of the invention, tomato ERFs according to the invention are proteins encoded by a gene localized on the genome close to at least one genetic marker such as a QTL (Quantitative Trait Locus). A quantitative trait locus is a region of DNA that is associated with a particular phenotypic trait, since they are closely linked to the genes that underlie the trait in question. QTLs can be molecularly identified (for example, with PCR or AFLP) to help map regions of the genome that contain genes involved in specifying a quantitative trait.

The man skilled in the art knows how to realize the genetic mapping of the ERFs and associated QTLs on the tomato genome. This will allow the implementation of association genetics strategy in order to uncover putative co-localization of a particular ERF with fruit quality QTLs and therefore to identify candidate ERF genes that are key regulator of specific aspects of fruit ripening and quality.

The invention is also related to a plant cell and to plants having a different expression of at least one of the ERF according to the invention. This difference may be an increased or a decreased expression level of the gene, an increased or a decreased expression level of the protein, or an increased or a decreased activity of the protein.

Such modulation of expression of genes or proteins are well known by the man skilled in the art, and can be obtained by various means including but not limited to genetic manipulation, deletion of a gene, replacement of a native promoter with a stronger one, stabilization or destabilization of the encoded RNA messenger, stabilization, degradation or exportation of the encoded protein.

The modulation of the activity of a protein can also be achieved by various means known by the man skilled in the art, including but not limited to introduction of point mutations into the gene leading to the translation of a protein with a different level of activity than the native protein.

This difference of expression may be observed in all cells of the plant, or only in part of them. This different level of expression may be constitutive or induced at a specific time, by addition of inducers.

The plant cell according to the invention may have in particular a genetic alteration in the regulatory or in the coding sequence of at least one ERF according to the invention.

The genetic alteration may result of a selection process of naturally occuring mutations, or of a genetic manipulation of the plant.

The genetic alteration may be a mutation, a deletion or an overexpression of the gene encoding the ERF according to the invention.

Genetic alterations according to the invention may result in different phenotypes, depending on the ERF that is mutated, deleted or overexpressed.

In an embodiment of the invention, at least one ERF encoding gene has its expression enhanced. To obtain an increase of the expression of a gene encoding a specific ERF, the man skilled in the art knows different methods, and for example:
- Replacement of the endogenous promoter of said gene encoding an ERF with a stronger promoter;
- Introduction into the cell of an expression vector carrying said gene encoding a specific ERF;
- Introducing additional copies of said gene encoding a specific ERF into the chromosome.

In another embodiment of the invention, at least one ERF encoding gene has its expression inhibited. To obtain the attenuation of the expression of a gene, different methods exist, and for example:
- Introduction of a mutation into the gene, decreasing the expression level of this gene, or the level of activity of the encoded protein;
- Replacement of the natural promoter of the gene by a low strength promoter, resulting in a lower expression;
- Use of elements destabilizing the corresponding messenger RNA or the protein;
- Deletion of the gene if no expression is needed.

Genetic transformation of plants are now well known in the art, comprising introducing a new gene fragment in a plant cell and then regenerating a plant form the cell. The new gene fragment is preferably introduced with known techniques of particle bombardment and/or infection with a transformed Agrobacterium. Regeneration procedure are also well known in the art and documented for numerous plant today. The new gene fragment is preferably integrated into the plant cell genome. New techniques of homologous recombination and gene replacement are also known today to be effective in plant cells.

The term « transformation » refers to the incorporation of exogenous nucleic acid by a cell, this acquisition of new genes being transitory (if the vector carrying genes is cured) or permanent (in the case the exogenous DNA is integrated chromosomally).

The term « vector » refers to an extra-chromosomal element carrying genes or cassettes, that is usually in the form of a circular double-stranded DNA molecules, but may be a single strand DNA molecule, too. Both terms "vector" and "plasmid" are used indifferently.

The invention is also related to a plant containing at least one plant cell such as described above.

The term "plant" according to the invention means any plant, monocotyledonous (small leafs) or dicotyledonous (broad leafs) and particularly crops having a commercial interest in the agricultural industry, more particularly crops selected among the group consisting of acacia, alfalfa, aneth, apple, apricot, artichoke, arugula, asparagus, avocado, banana, barley, beans, beet, blackberry, blueberry, broccoli, brussels sprouts, cabbage, canola, cantaloupe, carrot, cassaya, cauliflower, celery, cherry, cilantro, citrus, clementine, coffee, corn, cotton, cucumber, Douglas fir, eggplant, endive, escarole, eucalyptus, fennel, figs, forest trees, gourd, grape, grapefruit, honey dew, jicama, kiwifruit, lettuce, leeks, lemon, lime, loblolly pine, mango, melon, mushroom, nut, oat, okra, onion, orange, an ornamental plant, papaya, parsley, pea, peach, peanut, pear, pepper, persimmon, pine, pineapple, plantain, plum, pomegranate, poplar, potato, pumpkin, quince, radiata pine, radicchio, radish, rapeseed, raspberry, rice, rye, sorghum, Southern pine, soybean, spinach, squash, strawberry, sugarbeet, sugarcane, sunflower, sweet potato, sweetgum, tangerine, tea, tobacco, tomato, turf, a vine, watermelon, wheat, yams, and zucchini.

In a preferred embodiment, said plants are selected among fruiting and flowering vegetables, including Armenian cucumber (Cucumis melo Flexuosus group) , Eggplant (Solanum melongena), Avocado (Persea americana), Bell pepper (Capsicum annuum), Bitter melon (Momordica charantia), Caigua (Cyclanthera pedata), Cape Gooseberry (Physalis peruviana), Cayenne pepper (Capsicum frutescens), Chayote (Sechium edule), Chili pepper (Capsicum annuum Longum group), Cucumber (Cucumis sativus), Globe Artichoke (Cynara scolymus), Luffa (Luffa acutangula, Luffa aegyptiaca), Malabar gourd (Cucurbita ficifolia), Parwal (Trichosanthes dioica), Perennial cucumber (Coccinia grandis), Pumpkin (Cucurbita maxima, Cucurbita pepo), Pattypan squash Snake gourd (Trichosanthes cucumerina), Squash (Cucurbita pepo), Sweetcorn (Zea mays), Sweet pepper (Capsicum annuum Grossum group), Tinda (Praecitrullus fistulosus), Tomato (Solanum lycopersicum), Tomatillo (Physalis philadelphica), Winter melon (Benincasa hispida), West Indian gherkin (Cucumis anguria), Zucchini (Cucurbita pepo), and more preferably tomato plants.

Preferred phenotypes of plants and particularly of vegetable plants, more particularly of tomato plants according to the invention, are presented below.

In a specific embodiment of the invention, a plant having a genetic alteration in one of the gene encoding for an ERF according to the invention shows an increased tolerance to viral, bacterial and/or a fungal infections.

An increased plant tolerance to viral infections may be in particular an increased tolerance to one of the following virus: Tomato mosaic virus (ToMV), Tomato spotted wilt virus (TSWV), Tobacco and tomato ringspot virus, Curly top virus (Curly top virus), Potato virus Y virus, Tomato bushy stunt virus, Tomato etch virus, Tomato fern leaf virus (TYLCV), Tomato mottle virus, Tomato necrosis virus, Tomato spotted wilt virus, Tomato yellow leaf curl virus, Tomato yellow top virus, Tomato bunchy top virus, Tomato planto macho virus, Aster yellows virus, Tomato big bud virus, Torrado virus, Marchito Virus.

The man skilled in the art knows how to determine if a plant is resistant or not to a specific virus, bacteria or fungus using screening method well known to the one skilled in the art of agronomy, agrochemistry and agriculture.

An increased plant tolerance to bacterial infections may be in particular an increased tolerance to one of the following bacterial infections: Bacterial canker (*Clavibacter michiganensis* subsp. *Michiganensis),* Bacterial speck (*Pseudomonas syringae* pv. *Tomato),* Bacterial spot (*Xanthomonas campestris* pv. *Vesicatoria),* Bacterial stem rot and fruit rot (*Erwinia carotovora* subsp. *Carotovora),* Bacterial wilt (*Ralstonia solanacearum*), Pith necrosis (*Pseudomonas corrugata*), Syringae leaf spot (*Pseudomonas syringae* pv. *Syringae)*.

An increased plant tolerance to fungal infections may be in particular an increased tolerance to one of the following fungal infections : Alternaria stem canker (*Alternaria alternata f.sp. lycopersici*), Anthracnose (*Colletotrichum coccodes , Colletotrichum dematium, Colletotrichum gloeosporioides, Glomerella cingulata*), Black mold rot *(Alternaria alternata, Stemphylium botryosum, Pleospora tarda, Stemphylium herbarum, Pleospora herbarum, Pleospora lycopersici, Ulocladium consortiale, Stemphylium consortiale*), Black root rot (*Thielaviopsis basicola, Chalara elegans*), Black shoulder (*Alternaria alternata*), Buckeye fruit and root rot (*Phytophthora capsici, Phytophthora drechsleri, Phytophthora parasitica*), Cercospora leaf mold (*Cercospora fuligena*), Charcoal rot (*Macrophomina phaseolina*), Corky root rot (*Pyrenochaeta lycopersici*), Didymella stem rot (*Didymella lycopersici*), Early blight (*Alternaria solani*), Fusarium crown and root rot (*Fusarium oxysporum f.sp. radicis-lycopersici*), Fusarium wilt (*Fusarium oxysporum f.sp. lycopersici*), Gray leaf spot (*Stemphylium botryosum f.sp. lycopersici, Stemphylium lycopersici, Stemphylium floridanum, Stemphylium solani*), Gray Mold (*Botrytis cinerea, Botryotinia fuckeliana*), Late blight (*Phytophthora infestans*), Leaf mold (*Fulvia fulva*), Phoma rot (*Phoma destructiva*), Powdery mildew (*Oidiopsis sicula, Leveillula taurica*), Pythium damping-off and fruit rot (*Pythium aphanidermatum, Pythium arrhenomanes, Pythium debaryanum, Pythium myriowlum, Pythium ultimum*), Rhizoctonia damping-off and fruit rot (*Rhizoctonia solani, Thanatephorus cucumeris*), Rhizopus rot (*Rhizopus stolonifer*), Septoria leaf spot (*Septoria lycopersici*), Sour rot (*Geotrichum candidum, Galactomyces geotrichum, Geotrichum klebahnii*), Southern blight (*Sclerotium rolfsii, Athelia rolfsii*), Target spot (*Corynespora cassiicola*), Verticillium wilt (*Verticillium albo-atrum, Verticillium dahliae*), White mold (S*clerotinia sclerotiorum, Sclerotinia minor*).

In another embodiment of the invention, a plant, particularly a vegetable plant, more particularly a tomato plant having a genetic alteration in at least one of the gene encoding for an ERF according to the invention, shows an increased tolerance to insects attacks and/or increased tolerance to nematodes.

The man skilled in the art knows how to determine the resistance of a plant to a specific insect or nematode using screening method well known to the one skilled in the art of agronomy, agrochemistry and agriculture.

Preferentially, the genetic alteration in the regulatory or coding sequence of a member of the ERF family leads to increased plant tolerance to insects attacks such as: acarians, aphids, white flies, trips, caterpillar, leaf miner.

Preferentially, the genetic alteration in the regulatory or coding sequence of a member of the ERF family leads to increased plant tolerance to Nematodes such as: Root-knot (*Meloidogyne* spp.), Sting (*Belonolaimus longicaudatus*), Stubby-root (*Paratrichodorus* spp., *Trichodorus* spp.).

In another embodiment of the invention, a plant, particularly vegetable plant, more particularly a tomato plant having a genetic alteration in at least one of the gene encoding for an ERF according to the invention, shows an increased tolerance to abiotic stresses.

Abiotic stresses are for example: chilling, freezing, drought, salinity of the growth substrate, presence of heavy metals, wind, dehydration, high CO2 content, acid soils, basic soils.

In another embodiment of the invention, a plant, particularly a vegetable plant, more particularly a tomato plant having a genetic alteration in at least one of the gene encoding for an ERF according to the invention, shows modifications and in particular improvement of fruit properties.

A genetic alteration in the regulatory or coding sequence of a member of the ERF family leading to modifications of fruit properties such as : colour, shape, weight, shelf-life, maturation, flesh texture, sugar content, vitamin content, anti-oxydant content, firmness, freshness, mineral content, absence of seeds (Seedless), absence of Jelly (all-flesh), aroma content, flavour, volatile compounds composition, organoleptic properties.

In another embodiment of the invention, a plant, particularly a vegetable plant, more particularly a tomato plant having a genetic alteration in at least one of the gene encoding for an ERF according to the invention, shows modifications of plant agronomic properties.

A genetic alteration in the regulatory or coding sequence of a member of the ERF family can lead to modifications of plant agronomic properties such as such as: plant vigour, precocity, inter-node distance, number of flowers, fertility of flowers, plant architecture, absence of axillary shoots, parthenocarpy, apomixes.

In another embodiment of the invention, a plant, particularly a vegetable plant, more particularly a tomato plant having a genetic alteration in at least one of the gene encoding for an ERF according to the invention, shows modifications of seed properties.

A genetic alteration in the regulatory or coding sequence of a member of the ERF family can lead to modifications of seed properties such as such as: germination, yield, weight, size, conservation.

The present invention also concerns a seed of a plant according to the invention, or comprising at least one plant cell according to the invention.

The present invention also concerns a method for obtaining a plant having new phenotypic characteristics, comprising introducing into a plant cell a genetic alteration in the regulatory or in the coding sequence of at least one gene encoding an ERF protein as define above and below and regenerating at least one fertile plant comprising said cell.

This genetic alteration leads in particular to a modification of the level of expression of at least one of the ERF encoding genes.

Preferably, the plant being regenerated is further crossed with another plant and seeds are harvested, said crossing step being eventually repeated at least one time.

The invention also concerns seeds and progenies of plants of the invention, wherein said seeds and progenies have a genetic alteration in the regulatory or in the coding sequence of a gene encoding an ERF protein as defined above and below.

### DRAWINGS

**Figure 1**: Complete amino acid sequences of the twenty-eight claimed tomato ERF proteins.
**Figures 2**: Structural features of different subclasses of tomato ERF.
**Figure 3**: Phylogenetic tree of *Arabidopsis Thaliana* and *Solanum lycopersicum* ERF proteins.
   A- Homology of the amino acid sequence of the whole protein.
   B- Homology of the amino acid sequence of the AP2/ERF domain only.
**Figure 4**: Expression pattern of the ERF in different plant tissue and organs. Quantitative RT-PCR of ERF transcript in total RNA samples extracted from Stem (St), Roots (R), Leaves (L), Flower (Fl), Early Immature Green Fruit (EIMG), Mature Green Fruit (MG), Breaker Fruit (B), Breaker + 2 days (B+2), Breaker + 7 days (B+7).
**Figure 5**: ERF activity on synthetic or native complex promoter assessed by transient expression in a single cell system. The fluorescence of a reporter gene was assessed by flux cytometrie. Regulation by ERFs of the following pomoters were assayed :
   (A) a synthetic promoter gene containing 4XGCC box,
   (B) an ethylene-inducible native promoter containing GCC box,
   (C) an ethylene-inducible native promoter lacking the GCC box.
   1 refers to the activity of the promoters in the presence of native ERF proteins,
   2 refers to the activity of the promoters in the presence of ERF proteins fused to the SRDX repressor domain.
**Figure 6**:
   A- Tomato transgenic lines over-expressing the gene encoding ERF. E2
   B- Tomato transgenic lines expressing a "repression version" of ERF. B3 compared to a wild type tomato.

### EXAMPLES

### Example 1 :

The spatio-temporal pattern of expression of twenty-four members of the tomato ERF gene family was established. Quantitative RT-PCR of ERF transcript in total RNA samples were realized on extracts from Stem (St), Roots (R), Leaves (L), Flower (Fl), Early Immature Green Fruit (EIMG), Mature Green Fruit (MG), Breaker Fruit (B), Breaker + 2 days (B+2), and Breaker + 7 days (B+7). Results are presented in figure 4 and summarized in table 1 below.

| | |
|---|---|
| Mainly expressed in the fruit | *A1, B2, C1, C3, C6, D2, D3, E4, F2, F5* |
| Mainly expressed in the flower organ | *A2, A3, E3, F1, F3, G1* |
| Mainly expressed in vegetative tissues | *C5*, *G2* |
| Constitutive expression | *B1, B3, C2, E1, E2, F4.* |

These results indicate that ten ERFs are preferentially expressed in the fruit, six ERFs in flower organ and two in vegetative tissues, while the remaining ERFs display a constitutive pattern of expression.

### Example 2 :

ERF activity on synthetic or native promoter, positionned before a reporter gene, was assessed by transient expression in a single cell system. The fluorescence of the reporter gene was assessed by flux cytometrie. Regulation by thirteen ERFs of the following promoters was assayed:
(A) a synthetic promoter gene containing 4XGCC box,
(B) an ethylene-inducible native promoter containing GCC box, Osmotine promoter,
(C) an ethylene inducible native promoter lacking the GCC box, E4 promoter.
   1 refers to the activity of the promoters in the presence of native ERF proteins,
   2 refers to the activity of the promoters in the presence of ERF proteins fused to the SRDX domain, a dominant repressing domain.

Results are presented in figure 5 and in table 2 below :

| | |
|---|---|
| Transcriptional activation of ERF responsive genes | A3, B3, C3, C4, E1 |
| Transcriptional inhibition of ERF responsive genes | F1, F2, F3, F4, F5 |
| ERF able to modulate the activity of ethylene responsive promoters lacking the GCC box | B3, C3, D2, F5 |

Inventors here show that some ERFs modulate positively or negatively the activity of promoters, and that surprisingly, three ERFs (B3, D2, F5) can also modulate the transcriptional activity of native ethylene-responsive promoters lacking the canonical GCC box (see lane C, E4 promoter,). ERF. C3 shows also this capacity (result not shown).

### Example 3 :

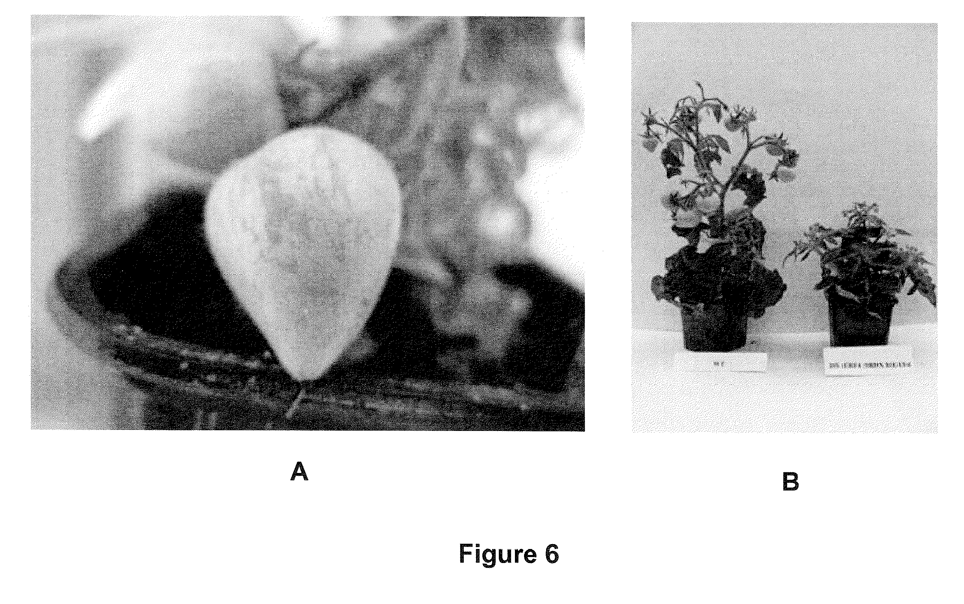

The physiological effects of the overexpression in tomato plants of the gene encoding ERF. E2 and a "repressing version" of ERF. B3, comprising a SRDX domain such as described above, were investigated. Figure 6 show that transgenic tomato expressing ERF.E2 have fruits with unusual color(A); and transgenic tomato plant expressing the "repressing version" of ERF.B3 is smaller than the corresponding wild-type tomato plant shown on the left (B).
This experiment demonstrates the impact of ERF expression on different properties of tomato plants.

## Claims

1. A tomato transcription factor of the Ethylene Responsive Factor family (ERF), having an amino acid sequence selected among the group consisting in
- a protein having an amino acid sequence as shown in one of SEQ ID NO 1 to NO 28,
- a variant thereof,
- a functional fragment thereof and
- a functional homologous sequence thereof.

2. A tomato transcription factor of the Ethylene Responsive Factor family (ERF), encoded by a DNA sequence selected among the group consisting in :
- a DNA fragment having a nucleotidic sequence as shown in one of SEQ ID N0 29 to NO 56
- a functional homologous sequence thereof.

3. The ERF of claim 1 or 2 wherein said factor presents at least one of the following structural features :
a. a nuclear localization signal
b. an acidic domain
c. an EAR domain
d. a MAP kinase phosphorylation site
e. a sequence "MCGGAII/L"
f. a sequence CMX-1
g. a glutamine rich region;
and combinations thereof.

4. The ERF of anyone of claims 1 to 3 wherein said factor is preferentially expressed :
a. in the fruit
b. in the flower organ
c. both in flowers and fruit
d. in vegetative tissues.

5. The ERF of one of claims 1 to 4, wherein said factor has a constitutive pattern of expression.

6. The ERF of one of claims 1 to 5, wherein said factor acts positively or negatively on the transcription of responsive genes.

7. The ERF of one of claims 1 to 6, wherein said protein is encoded by a gene localized on the genome close to at least one specific genetic marker (QTL).

8. A plant cell having a genetic alteration in the regulatory or in the coding sequence of a gene encoding an ERF protein as claimed in anyone of claims 1 to 7.

9. The plant cell of claim 8, wherein expression of at least one ERF as claimed in anyone of claims 1 to 6 is enhanced.

10. The plant cell according to claim 8, wherein expression of at least one ERF as claimed in anyone of claims 1 to 6 is partially or totally inhibited.

11. A plant containing at least one cell as claimed in any one of claims 8 to 10.

12. The plant of claim 11, wherein said plant has an increased tolerance to viral, bacterial and/or fungal infections and/or an increased tolerance to insects attacks and/or an increased tolerance to nematodes and/or an increased tolerance to abiotic stresses and/or modifications of fruit properties, and/or of plant agronomic properties, and/or of seed properties.

13. The plant cell as claimed in one of claims 8 to 10 or a plant as claimed in one of claims 11 to 12, wherein the plant is a tomato plant.

14. A seed of a plant as claimed in one of claims 11 or 12, wherein it comprises cells as claimed in one of claims 8 to 10.

15. A method for obtaining a plant having new phenotypic characteristics, comprising introducing into a plant cell a genetic alteration in the regulatory or in the coding sequence of at least one gene encoding an ERF protein as claimed in anyone of claims 1 to 7 and regenerating at least one fertile plant comprising said cell.

16. The method of claim 15, wherein the plant being regenerated is further crossed with another plant and seeds are harvested, said crossing step being eventually repeated at least one time.


SEQUENCE LISTING

<110>  INSTITUT NATIONAL POLYTECHNIQUE de TOULOUSE

<120>  NEW TOMATO ETHYLENE RESPONSE FACTORS AND USES THEREOF

<130>  67464 D26454

<160>  56

<170>  PatentIn version 3.3

<210>  1
<211>  234
<212>  PRT
<213>  solanum lycopersicom

<400>  1

Met Tyr Ser Asn Cys Glu Leu Glu Asn Asp Phe Ser Val Leu Glu Ser
1               5                   10                  15

Ile Arg Arg Tyr Leu Leu Glu Asp Trp Glu Ala Pro Leu Thr Ser Ser
            20                  25                  30

Glu Asn Ser Thr Ser Ser Glu Phe Ser Arg Ser Asn Ser Ile Glu Ser
        35                  40                  45

Asn Met Phe Ser Asn Ser Phe Asp Tyr Thr Pro Glu Ile Phe Gln Asn
    50                  55                  60

Asp Ile Leu Asn Glu Gly Phe Gly Phe Gly Phe Glu Phe Glu Thr Ser
65                  70                  75                  80

Asp Phe Ile Ile Pro Lys Leu Glu Ser Gln Met Ser Ile Glu Ser Pro
                85                  90                  95

Glu Met Trp Asn Leu Pro Glu Phe Val Ala Pro Leu Glu Thr Ala Ala
            100                 105                 110

Glu Val Lys Val Glu Thr Pro Val Glu Met Thr Thr Thr Thr Thr Lys
        115                 120                 125

Pro Lys Ala Lys His Tyr Arg Gly Val Arg Val Arg Pro Trp Gly Lys
    130                 135                 140

Phe Ala Ala Glu Ile Arg Asp Pro Ala Lys Asn Gly Ala Arg Val Trp
145                 150                 155                 160

Leu Gly Thr Tyr Glu Thr Ala Glu Asp Ala Ala Leu Ala Tyr Asp Lys
                165                 170                 175

Ala Ala Phe Arg Met Arg Gly Ser Arg Ala Leu Leu Asn Phe Pro Leu
            180                 185                 190

Arg Ile Asn Ser Gly Glu Pro Asp Pro Val Arg Val Gly Ser Lys Arg
        195                 200                 205

Ser Ser Met Ser Pro Glu His Cys Ser Ser Ala Ser Ser Thr Lys Arg
    210                 215                 220

Arg Lys Lys Val Ala Arg Gly Thr Lys Gln
225                 230

<210>  2
<211>  314
<212>  PRT
<213>  solanum lycopersicom

<400>  2

Met Asp Ser Ser Ser Leu Glu Met Ile Arg Gln His Leu Leu Asp Asp
1               5                   10                  15

Val Val Phe Met Glu Thr Cys Ser Ser Ser Ser Ser Ser Ser Leu Glu
            20                  25                  30

Thr Thr Ser Ser Thr Leu Tyr Ser Gln Thr Ser Ser Asn Ser Glu Ser
        35                  40                  45

Leu Glu Ser Leu Thr Ser Glu Ile Lys Leu Glu Ser Asn Phe Ser Val
    50                  55                  60

Tyr Pro Asp Phe Ile Asn Thr Pro Gln Ser Ser Asn Leu Glu Ser Val
65                  70                  75                  80

Ser Arg Phe Phe Asp Asn Ser Thr Ile Glu Phe Gln Ala Lys Pro Gln
                85                  90                  95

Lys Lys Arg Ser Phe Asn Asp Arg Lys Pro Ser Leu Asn Ile Ser Ile
            100                 105                 110

Pro Ser Val Lys Lys Thr Glu Glu Pro Lys Thr Gly Glu Val Lys Thr
        115                 120                 125

Gly Glu Pro Lys Thr Glu Glu Pro Lys Thr Gly Glu Val Lys Thr Glu
    130                 135                 140

Tyr Ser Val Lys Glu Lys Met Val Glu Asn Ser Glu Lys Lys Arg Tyr
145                 150                 155                 160

Arg Gly Val Arg Gln Arg Pro Trp Gly Lys Phe Ala Ala Glu Ile Arg
                165                 170                 175

Asp Pro Thr Arg Lys Gly Thr Arg Val Trp Leu Gly Thr Phe Asp Thr
            180                 185                 190

Ala Met Asp Ala Ala Met Ala Tyr Asp Arg Ala Ala Phe Arg Leu Arg
        195                 200                 205

Gly Ser Lys Ala Ile Leu Asn Phe Pro Leu Glu Val Ser Asn Phe Lys

210                 215                 220

Gln Glu Asn His Glu Ile Glu Lys Asn Val Val Asn Leu Asn Ser Asn
225                 230                 235                 240

Thr Asn Ser Cys Gly Lys Arg Val Arg Gly Glu Met Glu Asn Asp Asp
                245                 250                 255

Gly Ile Val Met Lys Lys Glu Val Lys Arg Glu Gln Met Val Ala Thr
            260                 265                 270

Pro Leu Thr Pro Ser Asn Trp Ser Ser Ile Trp Asp Cys Gly Asn Gly
        275                 280                 285

Lys Gly Ile Phe Glu Val Pro Pro Leu Ser Pro Leu Ser Pro His Ser
    290                 295                 300

Asn Phe Gly Tyr Ser Gln Leu Leu Val Ser
305                 310

<210>  3
<211>  244
<212>  PRT
<213>  solanum lycopersicom

<400>  3

Met Gly Ser Pro Gln Glu Thr Cys Thr Ser Leu Asp Leu Ile Arg Gln
1               5                   10                  15

His Leu Phe Asp Glu Ser Leu Asp Gln Thr Cys Phe Ser Phe Glu Thr
            20                  25                  30

Thr Gln Thr Ser Asn Leu Asp Asp Ile Ala Ser Phe Phe Asn Ala Thr
        35                  40                  45

Ser Lys Thr Glu Tyr Asp Gly Phe Phe Glu Phe Glu Ala Lys Arg His
    50                  55                  60

Val Ile Arg Ser Asn Ser Pro Lys Gln Ser Asn Leu Arg Glu Arg Lys
65                  70                  75                  80

Pro Ser Leu Asn Val Ala Ile Pro Ala Lys Pro Val Val Val Val Glu
                85                  90                  95

Asn Val Glu Ile Glu Lys Lys His Tyr Arg Gly Val Arg Gln Arg Pro
            100                 105                 110

Trp Gly Lys Phe Ala Ala Glu Ile Arg Asp Pro Asn Arg Lys Gly Thr
        115                 120                 125

Arg Val Trp Leu Gly Thr Phe Asp Thr Ala Val Asp Ala Ala Lys Ala
    130                 135                 140

Tyr Asp Arg Ala Ala Phe Lys Leu Arg Gly Ser Lys Ala Ile Leu Asn
145                 150                 155                 160

Phe Pro Leu Glu Val Ala Asn Phe Lys Gln Gln Asn Asp Glu Thr Lys
                165                 170                 175

Thr Glu Met Lys Ser Ser Gly Ser Lys Arg Val Arg Gly Glu Thr Glu
            180                 185                 190

Glu Leu Val Ile Lys Lys Glu Arg Lys Ile Glu Glu Glu Arg Val Leu
        195                 200                 205

Pro Thr Ala Ala Ala Pro Leu Thr Pro Ser Ser Trp Ser Thr Ile Trp
    210                 215                 220

Asp Glu Lys Gly Ile Phe Glu Val Pro Pro Leu Ser Pro Leu Ser Gln
225                 230                 235                 240

Leu Val Met Ile



<210>  4
<211>  233
<212>  PRT
<213>  solanum lycopersicom

<400>  4

Met Glu Ser Ser Ser Pro Lys Thr Gln Tyr Pro Asn Phe Asn Phe Phe
1               5                   10                  15

Gln Asp Gln Ser Ser Leu Pro Trp Asn Asp Gln His Phe Leu Asp Glu
            20                  25                  30

Tyr Leu Thr Asn Ile Asp Gln Asn Asn Asp His Ser Leu Pro Gly Ser
        35                  40                  45

Thr Cys Ser Phe Leu Thr Ser Lys Glu Ser Tyr Arg Arg Glu Val Ser
    50                  55                  60

Ser Ser Asn Leu His Gln Leu Pro Arg Ser Trp Ser Ser Ser Asn Asp
65                  70                  75                  80

Thr Asn Ser Ser Lys Glu Ser Asn Asn Arg His Glu Ile Glu Glu Val
                85                  90                  95

Thr Ser His His His Asp Lys Asn Asn Ser Thr Lys His Tyr Ile Gly
            100                 105                 110

Val Arg Lys Arg Pro Trp Gly Lys Tyr Ala Ala Glu Ile Arg Asp Ser
        115                 120                 125

Thr Arg Asn Gly Ile Arg Val Trp Leu Gly Thr Phe Asn Thr Cys Glu
    130                 135                 140

Glu Ala Ala Leu Ala Tyr Asp Gln Ala Ala Leu Thr Met Arg Gly Pro
145                 150                 155                 160

Leu Ala Leu Leu Asn Phe Pro Met Asp Lys Val Arg Glu Ser Leu Glu
                165                 170                 175

Asn Ile Lys Tyr Ile Cys Glu Asp Gly Ile Ser Pro Ala Ala Val Leu
            180                 185                 190

Lys Ala Thr Asn Lys Met Arg Arg Val Lys His Lys Arg Asn Arg Lys
        195                 200                 205

Lys Arg Asn Val Leu Val Phe Glu Asp Leu Gly Ala Glu Leu Leu Glu
    210                 215                 220

Glu Leu Leu Thr Ser Thr Ser Ser Asn
225                 230

<210>  5
<211>  193
<212>  PRT
<213>  solanum lycopersicom

<400>  5

Met Asp Tyr Ser Ser Arg Asp Asp Leu Leu Phe His Tyr Asn Ser Leu
1               5                   10                  15

Pro Phe Asn Val Asn Asp Thr Gln Asp Met Leu Leu Tyr Asn Leu Val
            20                  25                  30

Ala Glu Gly Ser Ser Gln Glu Thr Val Asn Ser Ser Ser Ser Tyr Gly
        35                  40                  45

Ile Lys Glu Glu Glu Val Thr Ser Tyr Glu Glu Glu Arg Lys Asp Lys
    50                  55                  60

Asn Tyr Arg Gly Val Arg Lys Arg Pro Trp Gly Lys Tyr Ala Ala Glu
65                  70                  75                  80

Ile Arg Asp Ser Thr Arg Asn Gly Val Arg Val Trp Leu Gly Thr Phe
                85                  90                  95

Asp Asn Ala Glu Glu Ala Ala Leu Ala Tyr Asp Gln Ala Ala Phe Ala
            100                 105                 110

Met Arg Gly Ser Met Ala Ile Leu Asn Phe Pro Val Glu Ile Val Lys
        115                 120                 125

Glu Ser Leu Asn Glu Met Lys Cys Arg Phe Asp Gly Asn Cys Ser Pro
    130                 135                 140

Val Ile Glu Leu Lys Lys Arg Tyr Ser Met Arg Arg Lys Ser Val Ser
145                 150                 155                 160

Arg Lys Asn Arg Ala Arg Lys Asp Val Val Val Phe Glu Asp Leu Gly
                165                 170                 175

Ala Glu Tyr Leu Glu Glu Leu Leu Ile Ser Ser Glu Ser Ile Thr Asn
            180                 185                 190

Trp

<210>  6
<211>  264
<212>  PRT
<213>  solanum lycopersicom

<400>  6

Met Arg His Ser Leu Lys Met Thr Thr His His Val Glu Asn Asn Asn
1               5                   10                  15

Gln Glu Gln Asp Gln Val Ala Cys Glu Glu Ile Leu Glu Asn Val Trp
            20                  25                  30

Ala Asn Phe Ile Ser Lys Asn Asp Gln Asn Ser Gln Lys Val Thr Asn
        35                  40                  45

Glu Tyr Cys Cys Glu Gln Tyr Trp Glu Gln Leu Pro Ile Leu Glu Arg
    50                  55                  60

Leu Pro Ser Leu Gly Arg Trp Ile Ser Met Gly Ala Glu Thr Trp Glu
65                  70                  75                  80

Asp Ile Leu Asn Gly Ile Ile Ile Pro Ser His Asn Asn Glu Asn Ser
                85                  90                  95

Asn Asp Glu Ser Thr Cys Lys Asp Val Val Asn Val Glu Lys Lys Glu
            100                 105                 110

Glu Lys Lys Lys Met Val His Tyr Arg Gly Val Arg Arg Arg Pro Trp
        115                 120                 125

Gly Lys Tyr Ala Ala Glu Ile Arg Asp Ser Ser Arg Lys Gly Ala Arg
    130                 135                 140

Val Trp Leu Gly Thr Phe Ser Thr Ala Glu Glu Ala Ala Met Ala Tyr
145                 150                 155                 160

Asp Lys Ala Ala Leu Arg Ile Arg Gly Pro Lys Ala Tyr Leu Asn Phe
                165                 170                 175

Pro His Glu Met Val Ala Gln Ala Ile Gly Ile Ser Asn Gly Pro Cys
            180                 185                 190

Glu Lys Glu Trp Thr Phe Ser Ser Ser Ser Gln Tyr Asn Ser Arg Lys

195                 200                 205

Arg Val Ser Arg Asp Trp Asn Met Tyr Glu Asn Leu Asp Glu Ile Asn
    210                 215                 220

Gln Leu Pro Met Glu Lys Lys Ile Met Arg Ser Met Glu Glu Asp Leu
225                 230                 235                 240

Phe Asn Asp Leu Asp Ile Leu Glu Phe Glu Asp Leu Gly Ser Asp Tyr
                245                 250                 255

Leu Asp Ser Leu Leu Ser Ser Leu
            260

<210>  7
<211>  303
<212>  PRT
<213>  solanum lycopersicom

<400>  7

Met Cys Asn Ile Val His Tyr Lys Val Ala Asn Ser Asn Asp Asn Arg
1               5                   10                  15

Ser Ser Arg Gln Asp Asp Glu Gly Ile Asn Val Phe Asn Thr Met Phe
            20                  25                  30

Gln Gly Asn Ile Asn Arg Glu Glu Glu Met Ser Val Met Val Ser Ala
        35                  40                  45

Leu Thr Arg Val Val Val Gly Asn His Pro Ser Glu Asn Ile Glu Asn
    50                  55                  60

His His Gln Asn Asn Thr Leu Ile Ser Arg Gly Val Gly Glu Lys Arg
65                  70                  75                  80

Gly Arg Asp Glu Val Leu Leu His Gly Thr Asn Ser Ser His Met Ile
                85                  90                  95

Leu Ser Ser Gly Gly Glu Gly Ser Ser Ile Arg Thr Thr Arg Glu Ala
            100                 105                 110

Thr Phe Ile Tyr Thr Asn Ser Thr Asn Asn Ser Ile Ile Asp Glu Ser
        115                 120                 125

Val Asn Asn Gln Val Arg Arg Arg Tyr Arg Gly Val Arg Gln Arg Pro
    130                 135                 140

Trp Gly Lys Trp Ala Ala Glu Ile Arg Asp Pro Tyr Lys Ala Ala Arg
145                 150                 155                 160

Val Trp Leu Gly Thr Phe Asp Thr Ala Glu Gly Ala Ala Arg Ala Tyr
                165                 170                 175

Asp Glu Ala Ala Leu Thr Phe Arg Gly Ser Lys Ala Lys Leu Asn Phe
            180                 185                 190

Pro Glu Asn Val Thr Leu Leu Val Pro Ser Ser Ile Gln Gln Pro Ile
        195                 200                 205

Tyr Ser Ser Pro Asp Pro Ala Ile Ser Pro Tyr Arg Ser Asn Phe Ile
    210                 215                 220

Ile Gly His Thr Ser Thr Glu Val Glu Pro Ile Leu His Thr Asn Pro
225                 230                 235                 240

Ser Asn Phe Ile Glu Pro Ile Ala His Thr Ser Ser Leu Tyr Arg Ser
                245                 250                 255

Asn Phe Ile Glu Arg Asn His His Met Val Gln Gln Glu Pro Tyr Phe
            260                 265                 270

Gln Ala Gly Ser Thr Ser Gly Gly Ser Asp Phe His Gln Thr Thr Asn
        275                 280                 285

Ser Ser Asn Ser Ser Ile Tyr Asp His Pro Ser Ser Ser Ser Gly
    290                 295                 300

<210>  8
<211>  367
<212>  PRT
<213>  solanum lycopersicom

<400>  8

Met Cys Phe Leu Lys Val Ala Asn Ser Arg Lys Ser Ser Glu Phe Val
1               5                   10                  15

Arg Phe Thr Asp Thr Asp Asp Thr Gln Thr Thr Ala Val Thr Ala Ile
            20                  25                  30

Gly Gly Gly Val Glu Gly Gly Gly Gln Phe Asp Tyr Ser Met Tyr Ser
        35                  40                  45

Gly Tyr Cys Asp Ser Gln Ala Arg Asp Met Ser Glu Met Val Thr Glu
    50                  55                  60

Phe Thr Arg Val Val Ser Gly Gln Asp Tyr Arg Pro Asp Thr Arg Cys
65                  70                  75                  80

Tyr Ser Val Asn Ser Pro Ser Pro Ala Tyr Ser Ser Ser Ser Ser Gly
                85                  90                  95

Ser Arg Ala Gly Leu Lys Arg Ser Arg Asp Gln Gln Glu Phe Gly Thr
            100                 105                 110

Gly Leu Ser Ser Ser Ser Ser Val Lys Ile Glu Glu Ala Thr Ser Met
        115                 120                 125

Val Ala Pro Ile Pro Ala Phe Thr Thr Thr Ile Thr Thr Thr Thr Thr
    130                 135                 140

Thr Gly Glu Gly Ser Ser Glu Glu Thr Gly Gly Asp Arg Arg Arg Lys
145                 150                 155                 160

Tyr Arg Gly Val Arg Gln Arg Pro Trp Gly Lys Trp Ala Ala Glu Ile
                165                 170                 175

Arg Asp Pro His Lys Ala Ala Arg Val Trp Leu Gly Thr Phe Asp Thr
            180                 185                 190

Ala Glu Ala Ala Ala Arg Ala Tyr Asp Glu Ala Ala Leu Arg Phe Arg
        195                 200                 205

Gly Asn Arg Ala Lys Leu Asn Phe Pro Glu Asn Ala Arg Leu Ser Ser
    210                 215                 220

Leu Pro Gln Thr Gln Asn Thr Val Thr Ser Thr Ile Ser Asn Pro Ser
225                 230                 235                 240

Pro Leu Ile Ala Gln Pro Thr Ser Phe Leu Asn Pro Ile Gln Ser Ser
                245                 250                 255

Asp Thr Thr Arg Asp Tyr Trp Glu Tyr Ser Gln Leu Leu Gln Asn Pro
            260                 265                 270

Gly Asp Phe Thr Asp Gln Gln Pro Ser Asn Leu Leu Glu Gln Met Phe
        275                 280                 285

Val Ala Ser Ser Met Ala Met Leu His Ser Asn Thr Leu Pro Leu Ile
    290                 295                 300

Ser Ser Ser Ser Ser Leu Ala Thr Ser Ala Thr Ser Ser Thr Ser Tyr
305                 310                 315                 320

Pro Leu Leu Phe Ser Ser Tyr Tyr Thr Pro Gln Thr Asn Gln Ile Gln
                325                 330                 335

Gly Thr Asn Thr Ser Ser Thr Ser Thr Thr Ser Ser Ser Ser Phe Ser
            340                 345                 350

Thr Thr Phe Trp Ser Ser Ser Ser Gln Tyr Pro Pro Ser Ser Ser
        355                 360                 365

<210>  9
<211>  295
<212>  PRT
<213>  solanum lycopersicom

<400>  9

Met His Trp Leu Asn Lys Arg Phe Arg Gln Glu Ala Gly Met Asn Ser
1               5                   10                  15

Asn Ser Asn Ser Leu Gln Asn Asn Asn Gln Phe Gln Gln Gln Gln Pro
            20                  25                  30

Arg Leu Thr Gly Asp Glu Glu Tyr Ser Val Met Val Ala Thr Leu Lys
        35                  40                  45

Asn Val Ile Asn Gly Asn Ile Pro Thr Gln Asn Tyr Gln Glu Phe Asn
    50                  55                  60

Val Phe Ser Pro Tyr Asn Tyr Ser Thr Ala Thr Thr Thr Thr Asn Val
65                  70                  75                  80

Thr Ser Ser Ser Ser Pro Ser Thr Ser Met Ser Thr Ser Phe Glu Gln
                85                  90                  95

Val Leu Gly Val Ser Ala Glu Gln Glu Pro Cys Gln Phe Cys Arg Ile
            100                 105                 110

Gln Gly Cys Leu Gly Cys Asp Ile Phe Gly Thr Thr Phe Ser Ser Ser
        115                 120                 125

Ser Ser Ala Pro Ala Ala Val Ala Ala Pro Val Ala Asp Asn Lys Lys
    130                 135                 140

Lys Ser Ser Ser Ser Ser Thr Ala Thr Val Ala Ile Ala Lys Lys Lys
145                 150                 155                 160

Lys Lys Asn Tyr Arg Gly Val Arg Gln Arg Pro Trp Gly Lys Trp Ala
                165                 170                 175

Ala Glu Ile Arg Asp Pro Arg Lys Ala Ala Arg Val Trp Leu Gly Thr
            180                 185                 190

Phe Thr Thr Ala Glu Glu Ala Ala Arg Ala Tyr Asp Lys Ala Ala Ile
        195                 200                 205

Glu Phe Arg Gly Pro Arg Ala Lys Leu Asn Phe Ser Phe Ala Asp Tyr
    210                 215                 220

Thr Val Asp Thr Gln Glu Gln Gln Ser Thr Leu Ser Ser Ser Pro Gln
225                 230                 235                 240

Gln Leu Pro Glu Glu Pro Gln Gln Ser Gln Thr Ala Asn Asn Asn Ser
                245                 250                 255

Asp Tyr Gly Asn Glu Ile Trp Asp Gln Leu Met Gly Asp Asn Glu Ile
            260                 265                 270

Gln Asp Trp Leu Thr Met Met Asn Phe Asn Gly Asp Ser Ser Asp Ser
        275                 280                 285

Gly Gly Asn Val His Ser Phe
    290                 295


<210>  10
<211>  185
<212>  PRT
<213>  solanum lycopersicom

<400>  10

Met Ser Pro Pro Leu Phe Arg Val Pro Glu Glu Thr Glu Arg Cys Gln
1               5                   10                  15

Tyr Cys Lys Ile Asn Gly Cys Leu Gly Cys Asn Tyr Phe Ala Thr Ser
            20                  25                  30

Ser Ala Ala Ala Gly Val Val Asn Asn Asn Lys Ala Leu Lys Ile Val
        35                  40                  45

Gly Lys Thr Lys Lys Lys Lys Lys Asn Tyr Arg Gly Val Arg Gln Arg
    50                  55                  60

Pro Trp Gly Lys Trp Ala Ala Glu Ile Arg Asp Pro Arg Arg Ala Ala
65                  70                  75                  80

Arg Val Trp Leu Gly Thr Phe Thr Thr Ala Glu Asp Ala Ala Arg Ala
                85                  90                  95

Tyr Asp Arg Ala Ala Ile Glu Phe Arg Gly Pro Arg Ala Lys Leu Asn
            100                 105                 110

Phe Ser Phe Thr Asp Tyr Thr Ser Ile Gln Gln His Asn Thr Thr Thr
        115                 120                 125

Pro Met Gln Val Leu Gln Gln Gln Gln Pro Ala Pro Ser Gln Leu Gln
    130                 135                 140

Gln Gly Ile Asn Thr Glu Glu Glu Glu Phe Trp Asp Gln Leu Met Asn
145                 150                 155                 160

Ser Asp Asn Glu Ile Gln His Tyr Leu Tyr Arg Glu Ser Ser Asp Ser
                165                 170                 175

Ala Asn Gly Tyr Ile Ala His Ser Phe
            180                 185


<210>  11
<211>  254
<212>  PRT
<213>  solanum lycopersicom

<400>  11

Met Cys Gly Gly Ala Ile Ile Ser Asp Tyr Asp Pro Ala Gly Ser Phe
1               5                   10                  15

Tyr Arg Lys Leu Ser Ala Arg Asp Leu Trp Ala Glu Leu Asp Pro Ile
            20                  25                  30

Ser Asp Tyr Trp Ser Ser Ser Ser Ser Ser Ser Thr Val Gly Lys Pro
        35                  40                  45

Asp Ser Ala Leu Ser Pro Val Thr His Ser Val Asp Lys Pro Asn Lys
    50                  55                  60

Ser Asp Ser Gly Lys Lys Gly Asn Lys Thr Val Lys Val Glu Lys Glu
65                  70                  75                  80

Lys Ser Ser Gly Pro Arg Pro Arg Lys Asn Lys Tyr Arg Gly Ile Arg
                85                  90                  95

Gln Arg Pro Trp Gly Lys Trp Ala Ala Glu Ile Arg Asp Pro Gln Lys
            100                 105                 110

Gly Val Arg Val Trp Leu Gly Thr Phe Asn Thr Ala Glu Asp Ala Ala
        115                 120                 125

Arg Ala Tyr Asp Glu Ala Ala Lys Arg Ile Arg Gly Asp Lys Ala Lys
    130                 135                 140

Leu Asn Phe Pro Ala Pro Ser Pro Pro Ala Lys Arg Gln Cys Thr Ser
145                 150                 155                 160

Thr Val Ala Ala Ala Asp Thr Pro Pro Ala Leu Leu Leu Glu Ser Ser
                165                 170                 175

Asp Asn Ser Pro Leu Met Asn Phe Gly Tyr Asp Val Gln Tyr Gln Ser
            180                 185                 190

Gln Thr Pro Tyr Tyr Pro Met Glu Met Pro Ile Val Ser Glu Asp Tyr
        195                 200                 205

Glu Leu Lys Glu Gln Ile Ser Asn Leu Glu Ser Phe Leu Glu Leu Glu
    210                 215                 220

Pro Ser Asp Gln Phe Ser Gly Ile Val Asp Ser Asp Pro Leu Asn Val
225                 230                 235                 240

Phe Leu Met Glu Asp Phe Ala Ser Thr His His Gln Phe Tyr
                245                 250

<210>  12
<211>  221
<212>  PRT
<213>  solanum lycopersicom

<400>  12

Met Arg Arg Gly Arg Ala Thr Pro Ala Ala Ala Ala Ala Ala Val Lys
1               5                   10                  15

Pro Asp Gly Ser Gly Gly Leu Lys Glu Ile Arg Phe Arg Gly Val Arg
            20                  25                  30

Lys Arg Pro Trp Gly Arg Phe Ala Ala Glu Ile Arg Asp Pro Trp Lys
        35                  40                  45

Lys Thr Arg Val Trp Leu Gly Thr Phe Asp Ser Ala Glu Asp Ala Ala
    50                  55                  60

Lys Ala Tyr Asp Ala Ala Ala Arg Thr Leu Arg Gly Pro Lys Ala Lys
65                  70                  75                  80

Thr Asn Phe Pro Leu Pro Met Tyr Ser Gln His His Gln Phe Asn Arg
                85                  90                  95

Ser Leu Asn Pro Asn Asp Arg Leu Val Asp Pro Arg Leu Tyr Ser Gln
            100                 105                 110

Glu Ala Pro Ile Ile Cys Gln Arg Pro Thr Ser Ser Ser Met Ser Ser
        115                 120                 125

Thr Val Glu Ser Phe Ser Gly Pro Arg Pro Pro Arg Gln Gln Thr Ala
    130                 135                 140

Val Leu Pro Ser Arg Lys His Pro Arg Ser Pro Pro Val Glu Pro Asp
145                 150                 155                 160

Asp Cys Arg Ser Asp Cys Asp Ser Ser Ser Ser Val Val Glu Asp Gly
                165                 170                 175

Asp Cys Glu Gly Gly Asn Asp Asn Ile Val Ser Ser Ser Leu Arg Asn
            180                 185                 190

Pro Leu Pro Phe Asp Leu Asn Phe Pro Pro Pro Met Asp Asp Val Tyr
        195                 200                 205

Ala Asn Ser Asn Asp Leu Tyr Cys Thr Ala Leu Cys Leu
    210                 215                 220

<210>  13
<211>  240
<212>  PRT
<213>  solanum lycopersicom

<400>  13

Met Arg Arg Ser Arg Ala Ala Ala Ala Ala Arg Gln Val Pro Ala Thr
1               5                   10                  15

Glu Val Pro Val Pro Ala Pro Val Ala Gly Glu His Asn Gly Ser Gly
            20                  25                  30

Gly Ser Lys Glu Ile Arg Phe Arg Gly Val Arg Lys Arg Pro Trp Gly
        35                  40                  45

Arg Phe Ala Ala Glu Ile Arg Asp Pro Trp Lys Lys Thr Arg Val Trp
    50                  55                  60

Leu Gly Thr Phe Asp Ser Ala Glu Asp Ala Ala Arg Ala Tyr Asp Ala
65                  70                  75                  80

Ala Ala Arg Thr Leu Arg Gly Pro Lys Ala Lys Thr Asn Phe Pro Leu
                85                  90                  95

Pro Ser Ser His His Leu Pro Pro Tyr Pro His His His Gln Phe Asn
            100                 105                 110

Gln Ser Ile Asn Pro Asn Asp Pro Phe Val Asp Ser Arg Leu Tyr Ser
        115                 120                 125

Gln Asp His Pro Leu Val Ser Gln Arg Pro Thr Ser Ser Ser Met Ser
    130                 135                 140

Ser Thr Val Glu Ser Phe Ser Gly Pro Arg Gln Pro Pro Arg Gln Gln
145                 150                 155                 160

Thr Ala Ala Ser Val Pro Ser Arg Lys Tyr Pro Arg Ser Pro Pro Val
                165                 170                 175

Val Pro Asp Asp Cys His Ser Asp Cys Asp Ser Ser Ser Ser Val Val
            180                 185                 190

Glu Asp Gly Glu Cys Asp Asn Asp Asn Ile Ala Ser Ser Ser Phe Arg
        195                 200                 205

Lys Pro Leu Pro Phe Asp Leu Asn Leu Pro Ala Pro Met Asp Asp Phe
    210                 215                 220

Ser Ala Asp Ala Tyr Ala Asp Asp Leu His Cys Thr Ala Leu Cys Leu
225                 230                 235                 240

<210>  14
<211>  198
<212>  PRT
<213>  solanum lycopersicom

<400>  14

Met Arg His Arg Lys Ser Ser Glu Leu Lys Arg Pro Gly Ser Asp Leu
1               5                   10                  15

Leu Gln Gln Pro Asp Ala Asp Pro Pro Arg Tyr Arg Gly Val Arg Lys
            20                  25                  30

Arg Pro Trp Gly Arg Phe Ala Ala Glu Ile Arg Asp Pro Ile Lys Lys
        35                  40                  45

Thr Arg Val Trp Leu Gly Thr Phe Asp Thr Ala Glu Asp Ala Ala Arg

50                  55                  60

Ala Tyr Asp Asp Ala Ala Arg Ala Leu Arg Gly Ala Lys Ala Lys Thr
65                  70                  75                  80

Asn Phe Asn Met Leu Pro Leu Thr Asp Asp Pro Tyr Asp Asp Glu Phe
                85                  90                  95

Glu Leu Phe Pro Asn Pro Arg Pro Ala Ser Ser Ser Met Ser Ser Thr
            100                 105                 110

Leu Glu Ser Ser Ser Gly Pro Arg Gly Gly Ser Ser Ser Lys Val Thr
        115                 120                 125

Arg Met Lys Ile Pro Arg Pro Val Arg Pro Met Glu Glu Cys Arg Ser
    130                 135                 140

Asp Cys Asp Ser Ser Ser Ser Val Val Asp Asp Arg Cys Asp Val Asp
145                 150                 155                 160

Gln Thr Ser Ser Phe Val Thr Lys Gln Pro Leu Pro Phe Asp Leu Asn
                165                 170                 175

Leu Pro Pro Pro Ser Asp Asn Asp Gly Val Asp Val Asp Asp Leu His
            180                 185                 190

Val Thr Ala Leu Cys Leu
        195

<210>  15
<211>  225
<212>  PRT
<213>  solanum lycopersicom

<400>  15

Met Ala Val Lys Asp Lys Ala Val Lys Gly Gly Asn Val Lys Val Asn
1               5                   10                  15

His Gly Val Lys Glu Val His Tyr Arg Gly Val Arg Lys Arg Pro Trp
            20                  25                  30

Gly Arg Tyr Ala Ala Glu Ile Arg Asp Pro Gly Lys Lys Ser Arg Val
        35                  40                  45

Trp Leu Gly Thr Phe Asp Thr Ala Glu Glu Ala Ala Lys Ala Tyr Asp
    50                  55                  60

Ala Ala Ala Arg Glu Phe Arg Gly Pro Lys Ala Lys Thr Asn Phe Pro
65                  70                  75                  80

Phe Pro Ala Glu Met Asn Asn Val Gly Asn Asn Asn Ser Gln Ser Pro
                85                  90                  95

Cys Gly Ser Ser Thr Val Glu Ser Ser Ser Gly Glu Thr Val Val His
            100                 105                 110

Ala Pro Asn Thr Arg His Ala Pro Leu Glu Leu Asp Leu Thr Arg Arg
        115                 120                 125

Leu Gly Ala Ala Ala Glu Gly Gly Arg Gly Gly Val Gly Tyr Pro Ile
    130                 135                 140

Leu His Gln Gln Pro Thr Val Ala Val Leu Pro Asn Gly Gln Pro Val
145                 150                 155                 160

Leu Leu Phe Asp Ser Met Trp Arg Pro Gly Val Val Ser Arg Pro Tyr
                165                 170                 175

Gln Val Val Pro Ala Thr Met Glu Phe Ala Gly Val Gly Ala Gly Val
            180                 185                 190

Val Thr Ser Val Ser Asp Ser Ser Ser Val Val Glu Glu Lys His Tyr
        195                 200                 205

Gly Lys Lys Gly Leu Asp Leu Asp Leu Asn Leu Ala Pro Pro Met Glu
    210                 215                 220

Val
225

<210>  16
<211>  293
<212>  PRT
<213>  solanum lycopersicom

<400>  16

Met Glu Ser Gln Lys Ile Lys Lys Lys Leu Val His Lys Thr Ile Thr
1               5                   10                  15

Thr Lys Tyr Asp His His Asn Lys Trp Thr Pro Lys Val Val Arg Ile
            20                  25                  30

Cys Tyr Thr Asp Cys Asp Ala Thr Asp Ser Ser Ser Asp Asp Asp Asp
        35                  40                  45

Asp Glu Arg Asn Arg Val Lys Lys Tyr Val Thr Glu Ile Lys Phe Glu
    50                  55                  60

Lys Lys Met Ala Ala Ala Asp Val Arg Lys Ser Leu Asn Ser Asn Lys
65                  70                  75                  80

Lys Lys Lys Lys Ala Ile Asp Leu Lys Arg Asp Glu Asn Val Lys Lys
                85                  90                  95

Phe Arg Gly Val Arg Gln Arg Pro Trp Gly Lys Trp Ser Ala Glu Ile
            100                 105                 110

Arg Asp Pro Val Lys Lys Thr Arg Val Trp Leu Gly Thr Phe Asp Thr
        115                 120                 125

Ala Glu Glu Ala Ala Met Lys Tyr Asn Ile Ala Ala Ile Gln Leu Arg
    130                 135                 140

Gly Ala Asp Ala Ile Ile Asn Phe Ile Glu Thr Pro Phe Pro Lys Glu
145                 150                 155                 160

Asn Ala Ile Thr Ser Val Ser Asp Tyr Asp Ser Thr Gly Glu Cys Glu
                165                 170                 175

Asn Leu Cys Ser Pro Thr Ser Val Leu Arg Gln Asn Asn Asn Asn Asn
            180                 185                 190

Asp Lys Asp Asn Glu Asp Ala Ile Ala Ile Asp Thr Lys Ile Met Asn
        195                 200                 205

Asp Glu Ser Lys Lys Met Glu Met Asp Glu Asn Gly Phe Met Phe Asp
    210                 215                 220

Asp Asn Leu Pro Leu Met Asp Gln Ser Phe Leu Lys Asp Phe Phe Asp
225                 230                 235                 240

Phe Arg Ser Pro Ser Pro Leu Met Asp Asp Val Leu Leu Pro Gly Phe
                245                 250                 255

Ser Asp Gly Met Gly Leu Leu Pro Glu Val Leu Ser Ile His Gly Asn
            260                 265                 270

Arg Met Leu Asp Glu Asp Leu Glu Thr Cys Lys Trp Ala Asn Asp Phe
        275                 280                 285

Phe Gln Asp Val Cys
    290

<210>  17
<211>  242
<212>  PRT
<213>  solanum lycopersicom

<400>  17

Met Tyr Gln Leu Pro Thr Ser Thr Glu Leu Thr Phe Phe Pro Ala Glu
1               5                   10                  15

Phe Pro Val Tyr Cys Arg Ser Ser Ser Phe Ser Ser Leu Met Pro Cys
            20                  25                  30

Leu Thr Glu Ser Trp Gly Asp Leu Pro Leu Lys Val Asn Asp Ser Glu
        35                  40                  45

Asp Met Val Ile Tyr Gly Phe Leu Gln Asp Ala Phe Ser Ile Gly Trp
    50                  55                  60

Thr Pro Ser Asn Leu Thr Ser Glu Glu Val Lys Leu Glu Pro Arg Glu
65                  70                  75                  80

Glu Ile Glu Pro Ala Met Ser Thr Ser Val Ser Pro Pro Thr Val Ala
                85                  90                  95

Pro Ala Ala Leu Gln Pro Lys Gly Arg His Tyr Arg Gly Val Arg Gln
            100                 105                 110

Arg Pro Trp Gly Lys Phe Ala Ala Glu Ile Arg Asp Pro Ala Lys Asn
        115                 120                 125

Gly Ala Arg Val Trp Leu Gly Thr Tyr Glu Ser Ala Glu Glu Ala Ala
    130                 135                 140

Leu Ala Tyr Asp Lys Ala Ala Phe Arg Met Arg Gly Thr Lys Ala Leu
145                 150                 155                 160

Leu Asn Phe Pro His Arg Ile Gly Leu Asn Glu Pro Glu Pro Val Arg
                165                 170                 175

Val Thr Val Lys Arg Arg Leu Ser Glu Ser Ala Ser Ser Ser Val Ser
            180                 185                 190

Ser Ala Ser Glu Ser Gly Ser Pro Lys Arg Arg Arg Lys Gly Val Ala
        195                 200                 205

Ala Lys Gln Ala Glu Leu Glu Val Glu Ser Arg Gly Pro Asn Val Met
    210                 215                 220

Lys Val Gly Cys Gln Met Glu Gln Phe Pro Val Gly Glu Gln Leu Leu
225                 230                 235                 240

Val Ser

<210>  18
<211>  201
<212>  PRT
<213>  solanum lycopersicom

<400>  18

Met Thr Lys Gln Asp Glu Gly Leu Thr Leu Glu Leu Ile Arg Gln His
1               5                   10                  15

Leu Leu Glu Asp Phe Thr Thr Thr Glu Ser Phe Ile Asp Ser Leu Asn
            20                  25                  30

Ser Cys Phe Ser Asp His Ile Ser Ser Ser Asp Asp Ile Ser Pro Val
        35                  40                  45

Phe Thr Ser Val Lys Thr Glu Pro Ser Thr Ser Asn Ser Leu Ser Asp

50                  55                  60

Ser Pro Asn Ser Ser Tyr Pro Asn Glu Pro Asn Ser Pro Ile Ser Arg
65                  70                  75                  80

Tyr Phe Asn Leu Arg Ser Asp Phe Pro Glu Phe Lys Ile Asp Ser Asp
                85                  90                  95

Thr Ile Leu Ser Pro Val Phe Asp Ser Ser Ala Gly Ser Asn Glu Asp
            100                 105                 110

Asn Asn Lys Lys Lys Asn Tyr Arg Gly Val Arg Arg Arg Pro Trp Gly
        115                 120                 125

Lys Phe Ala Ala Glu Ile Arg Asp Pro Ser Arg Lys Gly Ser Arg Ile
    130                 135                 140

Trp Leu Gly Thr Phe Asp Thr Asp Ile Asp Ala Ala Arg Ala Tyr Asp
145                 150                 155                 160

Cys Ala Ala Phe Lys Met Arg Gly Arg Lys Ala Ile Leu Asn Phe Pro
                165                 170                 175

Leu Asp Ala Gly Lys Ser Gly Ala Pro Ala Asn Val Gly Arg Lys Arg
            180                 185                 190

Arg Arg Glu Asn Lys Met Glu Leu Val
        195                 200

<210>  19
<211>  260
<212>  PRT
<213>  solanum lycopersicom

<400>  19

Met Cys Gly Gly Ala Ile Leu Ala Asp Ile Ile Pro Pro Arg Asp Arg
1               5                   10                  15

Arg Leu Ser Ser Thr Asp Leu Trp Pro Thr Asp Phe Trp Pro Ile Ser
            20                  25                  30

Thr Gln Asn Val Pro Leu Asn Pro Lys Arg Ala Arg Pro Ser Thr Gly
        35                  40                  45

Gly Glu Gln Met Lys Lys Arg Gln Arg Lys Asn Leu Tyr Arg Gly Ile
    50                  55                  60

Arg Gln Arg Pro Trp Gly Lys Trp Ala Ala Glu Ile Arg Asp Pro Arg
65                  70                  75                  80

Lys Gly Val Arg Val Trp Leu Gly Thr Phe Asn Thr Ala Glu Glu Ala
                85                  90                  95

Ala Arg Ala Tyr Asp Arg Glu Ala Arg Lys Ile Arg Gly Lys Lys Ala
            100                 105                 110

Lys Val Asn Phe Pro Asn Glu Asp Asp Asp His Tyr Cys Tyr Ser His
        115                 120                 125

Pro Glu Pro Pro Pro Leu Asn Ile Ala Cys Asp Thr Thr Val Thr Tyr
    130                 135                 140

Asn Gln Glu Ser Asn Asn Cys Tyr Pro Phe Tyr Ser Ile Glu Asn Val
145                 150                 155                 160

Glu Pro Val Met Glu Phe Ala Ser Tyr Asn Gly Ile Glu Asp Gly Gly
                165                 170                 175

Glu Glu Met Val Lys Asn Leu Asn Asn Arg Val Val Glu Glu Glu Glu
            180                 185                 190

Lys Thr Glu Asp Glu Val Gln Ile Leu Ser Asp Glu Leu Met Ala Tyr
        195                 200                 205

Glu Ser Leu Met Lys Phe Tyr Glu Ile Pro Tyr Val Asp Gly Gln Ser
    210                 215                 220

Val Ala Ala Thr Val Asn Pro Ala Ala Glu Thr Ala Val Gly Gly Gly
225                 230                 235                 240

Ser Met Glu Leu Trp Ser Phe Asp Asp Val Ser Arg Leu Gln Pro Ser
                245                 250                 255

Tyr Asn Val Val
            260

<210>  20
<211>  222
<212>  PRT
<213>  solanum lycopersicom

<400>  20

Met Ala Pro Lys Glu Lys Ile Gly Ala Val Thr Ala Met Ala Met Val
1               5                   10                  15

Asn Leu Asn Gly Ile Ser Lys Glu Val His Tyr Arg Gly Val Arg Lys
            20                  25                  30

Arg Pro Trp Gly Arg Tyr Ala Ala Glu Ile Arg Asp Pro Gly Lys Lys
        35                  40                  45

Ser Arg Val Trp Leu Gly Thr Phe Asp Thr Ala Glu Glu Ala Ala Arg
    50                  55                  60

Ala Tyr Asp Asn Ala Ala Arg Glu Phe Arg Gly Ala Lys Ala Lys Thr
65                  70                  75                  80

Asn Phe Pro Lys Leu Glu Met Glu Lys Glu Glu Asp Leu Lys Phe Ala
                85                  90                  95

Val Lys Asn Glu Ile Asn Arg Ser Pro Ser Gln Thr Ser Thr Val Glu
            100                 105                 110

Ser Ser Ser Pro Val Met Val Asp Ser Ser Ser Pro Leu Asp Leu Ser
        115                 120                 125

Leu Cys Gly Ser Ile Gly Gly Phe Asn His His Thr Val Lys Phe Pro
    130                 135                 140

Ser Ser Gly Gly Gly Phe Thr Gly Ser Val Gln Ala Val Asn His Met
145                 150                 155                 160

Tyr Tyr Ile Glu Ala Leu Ala Arg Ala Gly Val Ile Lys Leu Glu Thr
                165                 170                 175

Asn Arg Lys Lys Thr Val Asp Tyr Leu Gly Gly Gly Asp Ser Asp Ser
            180                 185                 190

Ser Thr Val Ile Asp Phe Met Arg Val Asp Val Lys Ser Thr Thr Ala
        195                 200                 205

Gly Leu Asn Leu Asp Leu Asn Phe Pro Pro Pro Glu Asn Met
    210                 215                 220

<210>  21
<211>  234
<212>  PRT
<213>  solanum lycopersicom

<400>  21

Met Asp Gln Gln Leu Pro Pro Thr Asn Phe Pro Val Asp Phe Pro Val
1               5                   10                  15

Tyr Arg Arg Asn Ser Ser Phe Ser Arg Leu Ile Pro Cys Leu Thr Glu
            20                  25                  30

Lys Trp Gly Asp Leu Pro Leu Lys Val Asp Asp Ser Glu Asp Met Val
        35                  40                  45

Ile Tyr Gly Leu Leu Lys Asp Ala Leu Ser Val Gly Trp Ser Pro Phe
    50                  55                  60

Asn Phe Thr Ala Gly Glu Val Lys Ser Glu Pro Arg Glu Glu Ile Glu
65                  70                  75                  80

Ser Ser Pro Glu Phe Ser Pro Ser Pro Ala Glu Thr Thr Ala Ala Pro
                85                  90                  95

Ala Ala Glu Thr Pro Lys Gly Arg His Tyr Arg Gly Val Arg Gln Arg
            100                 105                 110

Pro Trp Gly Lys Phe Ala Ala Glu Ile Arg Asp Pro Ala Lys Asn Gly
        115                 120                 125

Ala Arg Val Trp Leu Gly Thr Tyr Glu Thr Ala Glu Glu Ala Ala Ile
    130                 135                 140

Ala Tyr Asp Lys Ala Ala Tyr Arg Met Arg Gly Ser Lys Ala His Leu
145                 150                 155                 160

Asn Phe Pro His Arg Ile Gly Leu Asn Glu Pro Glu Pro Val Arg Val
                165                 170                 175

Thr Ala Lys Arg Arg Ala Ser Pro Glu Pro Ala Ser Ser Ser Gly Asn
            180                 185                 190

Gly Ser Met Lys Arg Arg Arg Lys Ala Val Gln Lys Cys Asp Gly Glu
        195                 200                 205

Met Ala Ser Arg Ser Ser Val Met Gln Val Gly Cys Gln Ile Glu Gln
    210                 215                 220

Leu Thr Gly Val His Gln Leu Leu Val Ile
225                 230

<210>  22
<211>  224
<212>  PRT
<213>  solanum lycopersicom

<400>  22

Met Ser Ser Pro Leu Glu Ile Asp Thr Ser Phe Ser His Ser Asn Leu
1               5                   10                  15

Leu Phe Leu Glu Asp Glu Ser Ser Trp Ser Asn Thr His Asp Pro Phe
            20                  25                  30

Val Asp Ile Asp Glu Tyr Leu Pro Ile Ile Ile Pro Cys Asn Asp Glu
        35                  40                  45

Glu Ile Val Val Glu Ser Ser Asn Thr Ser Thr Thr Thr Thr Thr Thr
    50                  55                  60

Thr Thr Ser Lys Val Ala Ser Ile Gln Asn Ile His His Asp Gln Glu
65                  70                  75                  80

Glu Val Thr Ser Ile Glu Lys Lys His Glu Asp Asp Gln Glu Lys His
                85                  90                  95

Tyr Ile Gly Val Arg Lys Arg Pro Trp Gly Lys Tyr Ala Ser Glu Ile
            100                 105                 110

Arg Asp Ser Thr Arg Asn Gly Ile Arg Val Trp Leu Gly Thr Phe Asp

115                 120                 125

Thr Ala Glu Glu Ala Ala Leu Ala Tyr Asp Gln Ala Ala Leu Ser Met
    130                 135                 140

Arg Gly Pro Trp Ser Leu Leu Asn Phe Pro Met Glu His Val Lys Lys
145                 150                 155                 160

Ser Leu Glu Asn Ile Glu Tyr Ser Cys Lys Asp Gly Leu Ser Pro Ala
                165                 170                 175

Ala Val Leu Lys Ala Thr His Lys Thr Arg Arg Val Lys His Lys Arg
            180                 185                 190

Ser Ser Arg Lys Lys Lys Asn Glu Asn Leu Glu Asn Val Phe Val Phe
        195                 200                 205

Gln Asp Leu Gly Val Glu Leu Leu Glu Glu Leu Leu Met Thr Ser Ser
    210                 215                 220

<210>  23
<211>  240
<212>  PRT
<213>  solanum lycopersicom

<400>  23

Met Asp Ser Ser Ser Ser Ser Ser Gln Phe Phe Tyr Ser Met Asn Ser
1               5                   10                  15

Asp Leu Asn Ser Ser Asp Ser Ser Tyr Glu Trp Ser Asn Phe Asn Thr
            20                  25                  30

Gln Ser Tyr Leu Pro Phe Asn Val Asn Asp Ser Glu Glu Met Leu Leu
        35                  40                  45

Phe Gly Val Leu Asn Ala Ala His Glu Glu Thr Thr Ser Glu Thr Val
    50                  55                  60

Thr Ser His Arg Val Lys Glu Glu Glu Val Thr Ser Glu Ser Glu Val
65                  70                  75                  80

Ile Glu Ala Ile Pro Ala Lys Glu Lys Ser Tyr Arg Gly Val Arg Arg
                85                  90                  95

Arg Pro Trp Gly Lys Phe Ala Ala Glu Ile Arg Asp Ser Thr Arg Asn
            100                 105                 110

Gly Val Arg Val Trp Leu Gly Thr Phe Asp Ser Ala Glu Asp Ala Ala
        115                 120                 125

Leu Ala Tyr Asp Gln Ala Ala Phe Ser Met Arg Gly Asn Ser Ala Ile
    130                 135                 140

Leu Asn Phe Pro Val Glu Thr Val Arg Asp Ser Leu Arg Asp Met Lys
145                 150                 155                 160

Cys His Val Asp Asp Asp Cys Ser Pro Val Val Ala Leu Lys Lys Arg
                165                 170                 175

His Ser Met Arg Lys Arg Ser Thr Asn Ser Lys Lys Val Asn Ser Ile
            180                 185                 190

Ser Lys Val Val Arg Glu Val Lys Met Glu Asn Val Asn Asn Val Val
        195                 200                 205

Val Phe Glu Asp Leu Gly Ala Asp Tyr Leu Glu Gln Leu Leu Ser Ser
    210                 215                 220

Ser Ser Ser Asp Gln Ser Ser Cys Asp Ala Thr Tyr Phe Ser Pro Trp
225                 230                 235                 240

<210>  24
<211>  161
<212>  PRT
<213>  solanum lycopersicom

<400>  24

Met Val Pro Thr Pro Gln Ser Asp Leu Pro Leu Asn Glu Asn Asp Ser
1               5                   10                  15

Gln Glu Met Val Leu Tyr Glu Val Leu Asn Glu Ala Asn Ala Leu Asn
            20                  25                  30

Ile Pro Tyr Leu Pro Gln Arg Asn Gln Leu Leu Pro Arg Asn Asn Ile
        35                  40                  45

Leu Arg Pro Leu Gln Cys Ile Gly Lys Lys Tyr Arg Gly Val Arg Arg
    50                  55                  60

Arg Pro Trp Gly Lys Tyr Ala Ala Glu Ile Arg Asp Ser Ala Arg His
65                  70                  75                  80

Gly Ala Arg Val Trp Leu Gly Thr Phe Glu Thr Ala Glu Glu Ala Ala
                85                  90                  95

Leu Ala Tyr Asp Arg Ala Ala Phe Arg Met Arg Gly Ala Lys Ala Leu
            100                 105                 110

Leu Asn Phe Pro Ser Glu Ile Val Asn Ala Ser Val Ser Val Asp Lys
        115                 120                 125

Leu Ser Leu Cys Ser Asn Ser Tyr Thr Thr Asn Asn Asn Ser Asp Ser
    130                 135                 140

Ser Leu Asn Glu Val Ser Ser Gly Thr Asn Asp Val Phe Glu Ser Arg
145                 150                 155                 160

Cys

<210>  25
<211>  372
<212>  PRT
<213>  solanum lycopersicom

<400>  25

Met Cys Gly Gly Ala Ile Ile Ser Asp Leu Val Pro Pro Ser Arg Ile
1               5                   10                  15

Ser Arg Arg Leu Thr Ala Asp Phe Leu Trp Gly Thr Ser Asp Leu Asn
            20                  25                  30

Lys Lys Lys Lys Asn Pro Ser Asn Tyr His Ser Lys Pro Leu Arg Ser
        35                  40                  45

Lys Phe Ile Asp Leu Glu Asp Glu Phe Glu Ala Asp Phe Gln His Phe
    50                  55                  60

Lys Asp Asn Ser Asp Asp Asp Asp Asp Val Lys Ala Phe Gly Pro Lys
65                  70                  75                  80

Ser Val Arg Ser Gly Asp Ser Asn Cys Glu Ala Asp Arg Ser Ser Lys
                85                  90                  95

Arg Lys Arg Lys Asn Gln Tyr Arg Gly Ile Arg Gln Arg Pro Trp Gly
            100                 105                 110

Lys Trp Ala Ala Glu Ile Arg Asp Pro Arg Lys Gly Ile Arg Val Trp
        115                 120                 125

Leu Gly Thr Phe Asn Ser Ala Glu Glu Ala Ala Arg Ala Tyr Asp Ala
    130                 135                 140

Glu Ala Arg Arg Ile Arg Gly Lys Lys Ala Lys Val Asn Phe Pro Asp
145                 150                 155                 160

Glu Ala Pro Val Ser Val Ser Arg Arg Ala Ile Lys Gln Asn Pro Gln
                165                 170                 175

Lys Ala Leu Arg Glu Glu Thr Leu Asn Thr Val Gln Pro Asn Met Thr
            180                 185                 190

Tyr Ile Ser Asn Leu Asp Gly Gly Ser Asp Asp Ser Phe Ser Phe Phe
        195                 200                 205

Glu Glu Lys Pro Ala Thr Lys Gln Tyr Gly Phe Glu Asn Val Ser Phe
    210                 215                 220

Thr Ala Val Asp Met Gly Leu Gly Ser Val Ser Pro Ser Ala Gly Thr
225                 230                 235                 240

Asn Val Tyr Phe Ser Ser Asp Glu Ala Ser Asn Thr Phe Asp Cys Ser
                245                 250                 255

Asp Phe Gly Trp Ala Glu Pro Cys Ala Arg Thr Pro Glu Ile Ser Ser
            260                 265                 270

Val Leu Ser Glu Val Leu Glu Thr Asn Glu Thr His Phe Asp Asp Asp
        275                 280                 285

Ser Arg Pro Glu Lys Lys Leu Lys Ser Cys Ser Ser Thr Ser Leu Thr
    290                 295                 300

Val Asp Gly Asn Thr Val Asn Thr Leu Ser Glu Glu Leu Ser Ala Phe
305                 310                 315                 320

Glu Ser Gln Met Lys Phe Leu Gln Ile Pro Tyr Leu Glu Gly Asn Trp
                325                 330                 335

Asp Ala Ser Val Asp Ala Phe Leu Asn Thr Ser Ala Ile Gln Asp Gly
            340                 345                 350

Gly Asn Ala Met Asp Leu Trp Ser Phe Asp Asp Val Pro Ser Leu Met
        355                 360                 365

Gly Gly Ala Tyr
    370

<210>  26
<211>  327
<212>  PRT
<213>  solanum lycopersicom

<400>  26

Met Cys Gly Gly Ser Ile Ile Ser Asp Tyr Ile Asp Pro Ser Arg Thr
1               5                   10                  15

Ser Arg Arg Leu Thr Ala Glu Phe Leu Trp Gly Arg Phe Asp Leu Gly
            20                  25                  30

Lys Lys Gln Lys Asn Pro Asn Asn Tyr His Ser Lys Ala Lys His Leu
        35                  40                  45

Arg Ser Glu Val Val Asp Asp Phe Glu Ala Asp Phe Gln Asp Phe Lys
    50                  55                  60

Glu Leu Ser Asp Asp Glu Asp Val Gln Val Asp Val Lys Pro Phe Ala
65                  70                  75                  80

Phe Ser Ala Ser Lys His Ser Thr Gly Ser Lys Ser Leu Lys Thr Val
                85                  90                  95

Asp Ser Asp Lys Asp Ala Ala Ala Asp Lys Ser Ser Lys Arg Lys Arg

            100                 105                 110

Lys Asn Gln Tyr Arg Gly Ile Arg Gln Arg Pro Trp Gly Lys Trp Ala
        115                 120                 125

Ala Glu Ile Arg Asp Pro Arg Lys Gly Val Arg Val Trp Leu Gly Thr
    130                 135                 140

Phe Asn Thr Ala Glu Glu Ala Ala Lys Ala Tyr Asp Ile Glu Ala Arg
145                 150                 155                 160

Arg Ile Arg Gly Lys Lys Ala Lys Val Asn Phe Pro Asp Glu Ala Pro
                165                 170                 175

Ala Pro Ala Ser Arg His Thr Val Lys Val Asn Pro Gln Lys Val Leu
            180                 185                 190

Pro Glu Glu Ser Leu Tyr Ser Leu Gln Ser Asp Ser Ala Ile Met Asn
        195                 200                 205

Ser Val Glu Asp Asp His Tyr Asp Ser Phe Gly Phe Phe Glu Glu Lys
    210                 215                 220

Pro Met Thr Lys Gln Tyr Gly Tyr Glu Asn Gly Ser Ser Ala Ser Ala
225                 230                 235                 240

Asp Thr Gly Phe Gly Ser Phe Val Pro Ser Ala Gly Gly Asp Ile Tyr
                245                 250                 255

Phe Asn Ser Asp Val Gly Ser Asn Ser Phe Glu Cys Ser Asp Phe Gly
            260                 265                 270

Trp Gly Glu Pro Cys Ser Arg Thr Pro Glu Ile Ser Ser Val Leu Ser
        275                 280                 285

Ala Ala Ile Glu Cys Asn Glu Ala Gln Phe Val Glu Asp Ala Asn Ser
    290                 295                 300

Gln Lys Lys Leu Lys Ser Cys Thr Asn Asn Pro Val Ala Asp Asp Gly
305                 310                 315                 320

Asn Pro Arg Tyr Tyr Gly Thr
                325

<210> 27
<211> 248
<212> PRT
<213> solanum lycopersicom

<400> 27

Met Thr Glu Asn Ser Val Pro Val Ile Lys Phe Thr Gln His Ile Val
1               5                   10                  15

Thr Thr Asn Lys His Val Phe Ser Glu His Asn Glu Lys Ser Asn Ser
            20                  25                  30

Glu Leu Gln Arg Val Val Arg Ile Ile Leu Thr Asp Ala Asp Ala Thr
        35                  40                  45

Asp Ser Ser Asp Asp Glu Gly Arg Asn Thr Val Arg Arg Val Lys Arg
    50                  55                  60

His Val Thr Glu Ile Asn Leu Met Pro Ser Thr Lys Ser Ile Gly Asp
65                  70                  75                  80

Arg Lys Arg Arg Ser Val Ser Pro Asp Ser Asp Val Thr Arg Arg Lys
                85                  90                  95

Lys Phe Arg Gly Val Arg Gln Arg Pro Trp Gly Arg Trp Ala Ala Glu
            100                 105                 110

Ile Arg Asp Pro Thr Arg Gly Lys Arg Val Trp Leu Gly Thr Tyr Asp
        115                 120                 125

Thr Pro Glu Glu Ala Ala Val Val Tyr Asp Lys Ala Ala Val Lys Leu
    130                 135                 140

Lys Gly Pro Asp Ala Val Thr Asn Phe Pro Val Ser Thr Thr Ala Glu
145                 150                 155                 160

Val Thr Val Thr Val Thr Glu Thr Glu Thr Glu Ser Val Ala Asp Gly
                165                 170                 175

Gly Asp Lys Ser Glu Asn Asp Val Ala Leu Ser Pro Thr Ser Val Leu
            180                 185                 190

Cys Asp Asn Asp Phe Ala Pro Phe Asp Asn Leu Gly Phe Cys Glu Val
        195                 200                 205

Asp Ala Phe Gly Phe Asp Val Asp Ser Leu Phe Arg Leu Pro Asp Phe
    210                 215                 220

Ala Met Thr Glu Lys Tyr Tyr Gly Asp Glu Phe Gly Glu Phe Asp Phe
225                 230                 235                 240

Asp Asp Phe Ala Leu Glu Ala Arg
                245

<210>  28
<211>  204
<212>  PRT
<213>  solanum lycopersicom

<400>  28

Met Ala Arg Ala Gln Gln Arg Tyr Arg Gly Val Arg Gln Arg His Trp
1               5                   10                  15

Gly Ser Trp Val Ser Glu Ile Arg His Pro Leu Leu Lys Thr Arg Ile
            20                  25                  30

Trp Leu Gly Thr Phe Glu Thr Ala Glu Asp Ala Ala Arg Ala Tyr Asp
        35                  40                  45

Glu Ala Ala Arg Leu Met Cys Gly Pro Arg Ala Arg Thr Asn Phe Pro
    50                  55                  60

Tyr Asn Pro Asn Met Pro Gln Thr Ser Ser Ser Lys Leu Leu Ser Thr
65                  70                  75                  80

Thr Leu Thr Ala Lys Leu His Lys Cys Tyr Met Ala Ser Leu Gln Met
                85                  90                  95

Thr Lys Thr Ser Pro Gln Gly Gln Lys Leu Ala Lys Asn Ala Thr Asn
            100                 105                 110

Val Gln Glu Ser Val Ile Asn Ser Tyr Lys Met Lys Gln Gln Met Leu
        115                 120                 125

Val Pro Lys Pro Ser Val Leu Leu Thr His His Asp His His Glu Glu
    130                 135                 140

Ala Lys Val Val Asn Leu Gly Val Gly Val Ile Arg Lys Val Glu Asp
145                 150                 155                 160

Gln Val Leu Glu Gly Ile Pro Gln Phe Val Lys Pro Leu Glu Asp Asp
                165                 170                 175

His Ile Glu Gln Met Ile Glu Glu Leu Leu Asp Tyr Gly Ser Ile Glu
            180                 185                 190

Leu Cys Ser Asn Val Val Pro Ser His Gln Ile Gln
        195                 200

<210>  29
<211>  705
<212>  DNA
<213>  solanum lycopersicom

<400>  29
atgtattcaa attgtgaact agaaaatgat ttttcagtac tcgaatcaat tagaagatac      60

ttacttgaag attgggaagc tccattaacg agctctgaaa actcaacatc ctcagagttc     120

agccggagca acagcattga atccaatatg tttagtaatt catttgatta tacacctgaa     180

atttttcaaa atgatattct taatgaagga tttggatttg gatttgaatt cgagacttct     240

gattttataa tccctaaatt agagtcacaa atgtcaatcg aatcacctga aatgtggaat     300

ttaccggaat ttgtggctcc attagagacg gcggcggagg tgaaagttga aacaccggtt     360

gagatgacaa ctacgacgac gaagccaaag gcaaagcatt atagaggtgt gagagtgagg     420

ccatggggga aattcgcggc ggaaattaga gatccggcga aaaatggagc acgagtttgg     480

ctcggtacat atgagacggc ggaggatgcg gcgttggctt acgacaaggc ggcttttcgc 540

atgcggggat cacgtgcatt gctgaatttt ccgttgagga ttaattccgg tgaaccggat 600

cctgttagag ttggatcgaa gagatcgtca atgtcgccgg agcattgttc atcggcgtcg 660

tcgacgaaga ggaggaagaa ggttgctcgt ggaacaaagc aataa 705

<210> 30
<211> 945
<212> DNA
<213> solanum lycopersicom

<400> 30
atggattctt cttcactaga aatgataaga caacatcttc ttgatgatgt tgttttcatg 60

gaaacttgtt cttcttcttc ttcttcttca ttagaaacaa caagtagtac actttattct 120

caaacctcat cgaattcgga atctttagaa tcattaacct ctgagatcaa acttgaaagc 180

aatttctctg tttatcctga tttcatcaat acacctcaaa gttcaaatct tgaatctgtc 240

tctcgttttt tcgataactc aactattgaa ttccaagcta aaccccaaaa gaaaagaagt 300

ttcaatgatc gaaaaccttc gttaaacatt tcgattcctt ctgttaagaa aacagaggaa 360

ccaaaaacag gggaagtaaa aacaggggaa ccaaaaacag aggagccaaa aacaggggaa 420

gtgaaaacag agtactctgt taaggagaaa atggtagaaa attcggagaa aaagcgatac 480

agaggagtga gacaaaggcc atgggggaaa tttgcagcgg agattcgtga cccaactaga 540

aaggggacac gagtttggtt aggaacattc gatactgcaa tggatgcagc catggcatat 600

gacagagcag catttaggct cagagggagt aaagcaatct tgaattttcc acttgaggta 660

agcaatttca agcaagaaaa tcatgagatt gagaaaaatg ttgtgaattt gaattcgaat 720

acgaattctt gtgggaaaag ggtgagaggg gaaatggaga atgatgacgg aattgtaatg 780

aagaaagagg tgaaaagaga acaaatggtg gcaactccat taacaccttc aaattggtct 840

tcaatttggg attgtggaaa tggaaaaggt atttttgaag tgccaccttt gtcaccatta 900

tcaccacatt caaattttgg ttattctcaa cttttggtat catag 945

<210> 31
<211> 735
<212> DNA
<213> solanum lycopersicom

<400> 31
atgggttctc cacaagagac ttgtacttca cttgatttga ttaggcaaca tctttttgat 60

gaatctctgg accagacttg tttctctttt gaaacaactc aaacttcaaa tcttgatgac 120

atcgcaagct tctttaatgc tacttcaaaa acagagtatg atggtttttt cgaatttgag 180

gcaaaacgac atgttatccg ttcaaattct ccgaaacaga gtaacttgag agaacggaag 240

ccatctctga acgtagcaat accggcgaag cctgttgttg ttgtagagaa cgttgagatt 300

gagaagaagc attacagggg agttagacag aggccatggg ggaagtttgc agcggagatt 360

cgtgacccaa atagaaaggg gactcgagtt tggttaggaa catttgatac tgctgtggat 420

gcggcaaagg catatgacag ggcagcgttt aagcttagag gaagcaaagc aatattgaat 480

ttcccactcg aagttgcaaa ctttaagcaa caaaatgatg agactaaaac agagatgaag 540

tcgtcaggca gtaaaagggt gagaggagaa acagaggaat tagtaatcaa gaaggaaagg 600

aaaatagaag aagaaagagt actcccaacg gcggcggctc cattaacacc gtcaagttgg 660

tcgacgattt gggatgaaaa gggtattttt gaggtgccac cattgtcacc attatctcag 720

cttgttatga tataa 735

<210> 32
<211> 702
<212> DNA
<213> solanum lycopersicom

<400> 32
atggaatctt catcccctaa aactcaatat ccaaatttca atttcttcca agatcaatca 60

tcattaccat ggaatgatca acatttctta gatgaatatt tgactaacat cgaccaaaac 120

aacgatcatt ctctaccagg aagtacttgt tcattcttaa cctcgaaaga aagttataga 180

cgggaagttt cctcctccaa cctacatcaa ttaccaagaa gttggtcatc ctcaaacgat 240

acgaattcct ctaaagaaag caataatcgt catgaaatcg aagaggtcac gtctcatcac 300

catgataaga ataactccac caaacactac ataggagtta gaaagagacc atggggaaaa 360

tatgcagcgg aaataaggga ttcaacaaga aatgggatta gggtttggtt aggaacattt 420

aatacttgtg aagaagctgc tttagcgtat gatcaagctg cacttacaat gagaggtcca 480

ttggcacttt taaattttcc aatggacaaa gtaagagaat cacttgaaaa tattaagtat 540

atttgtgaag atgggatttc accagctgct gttttaaagg caacaaataa aatgagacgt 600

gttaaacata agagaaatag gaagaagaga aatgttttgg tttttgaaga tttgggtgct 660

gaattgttag aggaactttt gacgagtact tcctctaatt ag 702

<210> 33
<211> 582
<212> DNA
<213> solanum lycopersicom

<400> 33
atggattatt catctcggga tgatcttctt tttcactata attcacttcc atttaacgtt 60

aacgatacac aagacatgtt actttataat cttgttgctg aaggatcatc gcaagaaaca 120

gtgaattcgt cgtctagcta tggaataaag gaagaggaag tgacctcata tgaagaagaa 180

agaaaagata agaattacag aggtgttaga aagaggccat ggggtaaata tgctgctgaa 240

attcgtgatt ctacgaggaa tggtgttcgt gtatggctag gtacatttga taatgctgaa 300

gaagctgcgt tagcgtatga tcaagctgca tttgctatga gaggttcgat ggccatactt 360

aattttcctg tggagatagt taaggaatcg ctaaatgaaa tgaagtgtag atttgatggt 420

aattgttcgc cggtgattga attgaagaag agatattcaa tgaggagaaa gagtgttagt 480

agaaaaaaca gagcaagaaa agatgttgtg gtttttgaag atttgggtgc tgagtattta 540

gaggaattgt tgatttcttc agaaagtatc acaaattggt ga 582

<210> 34
<211> 795
<212> DNA
<213> solanum lycopersicom

<400> 34
atgagacatt ctttaaagat gactactcat catgtggaaa ataataatca agaacaagat 60

caagtagcat gtgaagagat tctagagaat gtttgggcaa acttcatatc caagaatgat 120

caaaattctc aaaaggtgac aaatgaatat tgttgtgaac aatattggga acaacttcca 180

attcttgaaa gattaccaag cttaggaaga tggatatcaa tgggagctga aacttgggaa 240

gatattctca atggaattat tattccttct cataacaacg aaaattcaaa cgacgaatca 300

acatgcaaag atgttgtcaa cgttgagaag aaggaggaga agaagaagat ggtgcattat 360

agaggggtaa gaaggaggcc atggggaaaa tacgcggctg agataaggga ttcatcaaga 420

aaaggagcta gggtttggct agggacattt agtactgctg aagaagctgc tatggcttat 480

gacaaggcgg ctttgagaat tagaggtccc aaggcctatc ttaattttcc acatgaaatg 540

gttgctcaag ctataggaat atctaatggt ccttgtgaaa aagaatggac cttttcatca 600

tcaagtcaat ataattcaag gaaaagggta tcaagggatt ggaacatgta tgaaaatttg 660

gatgaaatta atcaattgcc tatggagaag aaaattatga gaagcatgga ggaggatctt 720

tttaatgact tggatatcct agagtttgag gatcttggaa gtgattattt ggatagttta 780

ttatcctctt tgtaa 795

<210> 35
<211> 912
<212> DNA
<213> solanum lycopersicom

<400> 35
atgtgtaata ttgttcacta caaggtggcg aattcgaatg ataacaggag tagtagacaa 60

gacgatgaag ggattaatgt gtttaatacg atgtttcaag ggaatattaa tagagaagaa 120

gaaatgtctg ttatggtttc tgcattaact cgtgttgttg ttggtaatca tcctagtgaa 180

aatatcgaaa atcatcatca aaataataca ttgatttcta ggggtgttgg agaaaaaaga 240

ggacgtgatg aagtattatt acatggaact aattcttctc atatgatatt atcatcaggt 300

ggtgaaggtt caagcattag gacaacaaga gaagcaacat tcatatacac taattcaaca 360

aacaatagca ttattgatga atctgttaat aatcaagtaa gacgacgata cagaggagtt 420

agacagaggc catgggggaa atgggcagct gaaataagag atccatataa agcagcgcgt 480

gtttggttag gtacttttga tactgctgaa ggggctgcta gagcttatga tgaagctgct 540

cttacattta gaggtagtaa agcaaaacta aatttcccag aaaatgttac attattagtg 600

ccttcttcaa ttcaacaacc catttactcc tcacccgacc cagccatttc tccgtatcgt 660

tccaatttta ttattggaca tacctctacg gaagtggagc ctatacttca caccaatcct 720

tcaaatttta ttgagcctat agctcacact tcatctctgt atcgttccaa ttttattgaa 780

cggaatcatc acatggtgca gcaggagccg tattttcagg caggtagtac tagtggtgga 840

agtgattttc atcaaactac aaattcttct aattcgtcaa tttatgatca cccttcttct 900

tcatctggat aa 912

<210> 36
<211> 1104
<212> DNA
<213> solanum lycopersicom

<400> 36
atgtgctttt taaaggtggc gaattcaaga aaatcaagtg aatttgttag atttacagat 60

acagatgata cacaaaccac cgccgtgact gcgatcggcg gcggtgttga aggcggtggt 120

cagtttgatt attcgatgta ttcagggtat tgtgattctc aggcgagaga tatgtcggag 180

atggtgacgg agtttacacg tgtggtatcg ggtcaggatt atcgacccga taccagatgt 240

tattcggtta attcaccgtc tccggcttat tcttcgtcca gctcgggttc tagagctgga 300

ctgaagagaa gccgtgatca acaagaattt ggaactgggt tgtcatcttc ttcctctgtt 360

aaaattgaag aagctacaag tatggttgca ccaattcccg ctttcacaac cacaatcaca 420

accacgacca caacaggtga gggttcgagc gaagaaacag gaggagatag gaggaggaaa 480

tacagaggtg tacgacaacg accatggggt aaatgggcag cggaaataag agatccacat 540

aaagccgcca gagtttggtt aggaacattc gatacagcag aagctgcagc aagagcatat 600

gatgaagctg cattgagatt tcgaggaaac agagcaaaac tcaacttccc tgagaacgcc 660

agattgtcat cgttaccaca aacacaaaat actgtaacgt caacaatctc caatccatcc 720

cctctaatag ctcaaccaac gtcgttcctc aatcctatcc agagttcaga tacaacaaga 780

gactactggg aatactcaca attgttgcaa aatccaggag attttacgga tcaacaacca 840

tcaaacttat tggaacaaat gttcgttgcc tcatcgatgg caatgttgca ttcaaacaca 900

ttgccattaa tatcttcgtc ttcatcgtta gctacatcag caacttcttc aacgtcatat 960

cccctgttat tttcgagtta ttacacacca caaactaatc aaattcaagg aaccaacaca 1020

agtagcacca gcaccactag cagctcaagt ttttctacaa cattttggag tagctctagc 1080

caatatcctc catcttctag ttaa 1104

<210> 37
<211> 888
<212> DNA
<213> solanum lycopersicom

<400> 37
atgcattggt taaataaaag atttagacaa gaagcaggaa tgaattcgaa ttcgaattcc 60

ctccaaaata acaatcaatt tcaacagcag caaccaaggc ttactggaga tgaagagtac 120

tctgttatgg ttgcaactct gaaaaatgtg atcaatggta atattccaac gcaaaattat 180

caagaattca atgtcttttc gccatataat tattctactg ccaccacaac cacgaatgtt 240

acttcttctt cttcgccttc tactagtatg tctactagtt tcgaacaagt attgggtgtc 300

tctgctgaac aagaaccttg tcaattttgc agaattcaag gttgtttagg ctgtgacatt 360

tttggtacca cattttcttc ttcttcttca gcgcctgctg ctgtggctgc tcctgttgct 420

gataataaga agaagagtag tagtagtagt acagctacag tcgcaatcgc gaagaaaaag 480

aagaagaatt acagaggagt gagacagagg ccatggggga aatgggcagc agaaattcgt 540

gatcctcgaa aagctgcacg tgtttggctg ggaactttca ctacggcaga agaagcagct 600

agagcttatg ataaagccgc cattgaattc aggggtccac gagctaaatt gaatttttca 660

tttgcggatt ataccgttga cactcaagaa caacagagca ctttatcttc ttcaccacaa 720

caattaccag aagagcctca gcaatcccag acagcgaata ataattccga ttatggaaat 780

gaaatttggg atcaattgat gggtgacaat gaaattcaag attggttgac catgatgaat 840

ttcaatggcg actcttctga ttctggcggg aatgttcaca gcttttaa 888

<210> 38
<211> 558
<212> DNA
<213> solanum lycopersicom

<400> 38
atgtcgccgc ccttgtttcg cgtaccggaa gaaacagagc gttgtcagta ctgcaaaata 60

aatggttgtt taggctgcaa ctattttgca acctcatcag ctgcagctgg tgttgttaac 120

aacaacaagg cattaaagat tgttgggaag acgaaaaaga agaagaagaa ttacagggga 180

gtgagacaga gaccatgggg aaaatgggca gcggaaatta gagatccaag aagggcagct 240

agagtatggc ttggaacatt tactacagct gaggacgcag ctagagctta tgacagagca 300

gctattgaat ttagaggtcc aagagctaag cttaatttct catttacaga ttacacttca 360

attcaacaac acaatactac tacacccatg caagtgctgc aacaacaaca accagctccc 420

tcgcagttac aacaaggaat aaacacagaa gaagaagagt tctgggatca attgatgaat 480

tcggacaatg aaattcaaca ttatctttat agagaatcat ctgattctgc taatggctat 540

attgctcata gcttctag 558

<210> 39
<211> 765
<212> DNA
<213> solanum lycopersicom

<400> 39
atgtgtggag gtgccataat ctccgattat gatcccgccg gaagcttcta ccggaaactt 60

tctgctcgtg acctctgggc tgagctggac cctatctccg actactggtc ctcttcttcc 120

tcatcctcaa ccgtcggaaa acctgattcc gctctgtcgc cggtgactca ctccgtcgat 180

aagccaaata aatcagattc cggcaaaaaa ggtaataaga ctgtgaaggt tgagaaggag 240

aagagtagtg gaccaaggcc aaggaagaac aagtacagag gaataagaca gaggccatgg 300

ggaaaatggg ctgctgagat tcgcgatcca cagaagggtg tacgcgtttg gcttggtaca 360

ttcaacacag cagaagatgc tgctagagcc tatgatgagg ctgctaagcg cattcgtggt 420

gataaggcta aactcaactt tccagcccca tcaccaccag ctaagcgaca gtgcactagc 480

actgtcgctg ctgctgatac accaccagca ctactccttg agagttctga caactctcct 540

ttgatgaact ttggatatga tgtccagtat cagagccaaa ctccctacta ccccatggaa 600

atgcccatag ttagtgaaga ttatgaactg aaggaacaga tttccaattt ggaatcgttc 660

ctggaattgg agccatctga tcaattttca gggatcgtcg attctgatcc tcttaatgtt 720

tttctgatgg aggactttgc ttcaactcat catcagttct actga 765

<210> 40
<211> 666
<212> DNA
<213> solanum lycopersicom

<400> 40
atgagaagag gcagagcaac tccggcggcg gcggcggcgg cggtgaagcc agatggatct 60

ggaggattga aggagattag gtttcgtgga gttcggaaga ggccatgggg gagatttgct 120

gcagagatta gagatccatg gaagaaaact agggtttggt taggtacttt tgattcagct 180

gaagatgctg ccaaagctta tgatgctgca gctcgaactc ttcgtggacc taaagctaaa 240

actaatttcc ctttacctat gtattctcag catcatcagt tcaatcgaag tttaaaccct 300

aatgatcggt tagttgaccc gagattgtac tcacaagaag ctccgatcat ttgtcaaaga 360

cctacatcga gcagtatgag tagtactgtg gaatcattca gtggaccgag accgccacgt 420

cagcaaacgg cggttttgcc ttcgagaaaa catcctagat cgccgccggt tgagccggat 480

gactgcagga gtgattgtga ctcatcgtct tctgttgttg aagatggtga ttgtgaaggg 540

ggaaatgaca acatcgtttc ttcatctctc agaaatccac tgcctttcga tctcaacttt 600

ccacctccga tggatgatgt ttatgctaat tcaaatgatc tttactgcac agcactatgt 660

ctttga 666

<210> 41
<211> 723
<212> DNA
<213> solanum lycopersicom

<400> 41
atgcggagaa gcagagcagc cgctgcggcg agacaagttc cggcgacgga agttccggta 60

ccggcaccgg tggccggaga acacaacgga tctggaggat ctaaggagat aaggttccgt 120

ggagttcgaa agagaccatg gggaagattt gcagcagaga ttagagatcc atggaagaaa 180

actagggttt ggttgggtac ttttgattct gctgaagatg ctgctcgtgc ttatgatgca 240

gcagctcgta ctcttcgtgg acctaaagct aaaactaatt tccctttacc ttcttctcat 300

catctacctc catatcctca ccatcatcag ttcaaccaaa gcatcaaccc taacgatccc 360

tttgtcgatt cccggttata ctctcaggac cacccattag tttcacagag acctacttca 420

agcagcatga gtagtacggt ggagtccttc agtggaccac ggcagccgcc gcggcagcag 480

acggcagctt ccgtgccttc cagaaagtat ccccggtcac cgcctgttgt cccggacgat 540

tgccatagcg actgtgactc atcgtcttct gtcgttgaag acggtgaatg tgataacgac 600

aacatcgctt cttcctcttt cagaaagccg ttgcctttcg atctaaactt accggcaccg 660

atggatgact tcagcgccga cgcatatgcc gatgatcttc actgcacagc actatgtctt 720

tga 723

<210>  42
<211>  597
<212>  DNA
<213>  solanum lycopersicom

<400>  42
atgcgccacc ggaagtcgtc ggagctgaaa agaccaggat ctgacctcct tcaacagcct      60

gacgccgacc cacctcgtta tcgaggtgtt cgtaaacggc catggggtag attcgcagca     120

gagattagag atccgattaa aaagactcga gtttggctgg gtacctttga cacagctgaa     180

gacgccgcac gcgcttacga cgatgctgca cgcgctctcc gtggagctaa ggcgaaaact     240

aatttcaata tgttacctct aacagatgat ccttatgatg atgagtttga gcttttcccc     300

aatccgagac cggcttctag cagtatgagc agtacgttgg aatcgtctag tgggcctcgt     360

ggcggatcga gtagtaaggt gacccggatg aagattcctc gcccagttcg tccgatggag     420

gaatgccgga gtgattgcga ttcgtcgtcg tctgtggtgg atgatcggtg tgatgttgat     480

caaacgtcat cgtttgtgac caaacaacct ctgccgttcg atctgaatct gccacctccg     540

tcggataacg atggagttga tgttgatgat ttgcacgtca ccgctttatg cctctaa        597

<210>  43
<211>  678
<212>  DNA
<213>  solanum lycopersicom

<400>  43
atggctgtga aagataaggc tgtgaaagga ggtaatgtga aagtgaatca tggagttaag      60

gaagttcact acagaggtgt aaggaagagg ccatggggtc gttacgctgc ggagattcgt     120

gacccgggta agaagagtcg ggtctggctg ggtacttttg atacggcgga ggaagcggct     180

aaggcttacg atgccgctgc cagagagttt cgtggaccta aagcgaagac gaatttcccc     240

tttccggcgg agatgaataa tgttggtaac aataacagtc agagcccgtg tgggagcagt     300

accgtggagt catccagcgg agaaacggtt gttcacgcgc ctaatacgcg acacgcgccg     360

ctggagctgg atctcacgcg ccgtctcggt gccgctgctg aaggtggacg tggaggtgtc     420

ggctacccga tcttacacca gcagccgacg gtggcggttc tgccgaacgg tcagccggtt     480

ttgttgtttg attctatgtg gagaccggga gttgttagta ggccgtatca ggttgtaccg     540

gcgacgatgg agtttgccgg tgtcggtgcc ggagttgtta ctagtgtgtc ggattcgtct     600

tccgttgtgg aagagaaaca ttatgggaaa aagggacttg atcttgatct taaccttgcg     660

ccacctatgg aagtttaa                                                   678

<210>  44
<211>  882
<212>  DNA
<213>  solanum lycopersicom

<400>  44
atggaatcac aaaaaatcaa aaagaaatta gtccacaaaa ctatcactac taagtatgat      60

catcacaaca agtggactcc taaagttgtt cggatttgtt acactgattg tgatgctact     120

gattcttcaa gcgacgacga tgacgacgag aggaatcgag tgaaaaaata cgttacagag     180

attaaatttg agaagaaaat ggctgctgca gatgtgagga aatcgttgaa ttcgaataag 240

aagaagaaga aagcgatcga tttgaagaga gatgagaatg ttaaaaagtt tcgcggtgtc 300

agacagaggc catggggaaa atggtctgcg gagattcgag atccggtgaa aaaaacgagg 360

gtttggttag gtacttttga taccgctgaa gaagcggcta tgaaatataa tatagccgct 420

attcaattgc gcggagctga tgctatcatt aattttattg agacaccttt cccaaaggaa 480

aatgcgatca cttcagtatc ggattatgat tccacagggg aatgtgaaaa cctctgttct 540

cccacctcag ttttgaggca gaataataac aataatgata aagataacga agatgcgatt 600

gcgattgata ctaaaattat gaacgatgag agcaaaaaaa tggaaatgga tgaaaatgga 660

tttatgtttg atgataattt gccattaatg gatcagagtt tccttaagga tttcttcgat 720

tttcgatccc cttctccatt gatggatgat gtattattac caggttttag cgatggaatg 780

ggattattac cagaagtgtt gagtattcat ggaaatagaa tgttggatga agatttggag 840

acttgtaagt gggcaaatga tttcttccaa gatgtttgtt ga 882

<210> 45
<211> 742
<212> DNA
<213> solanum lycopersicom

<400> 45
atgtatcaac ttcccacttc tactgagtta actttttttc cggcagaatt cccggtgtat 60

tgccggagtt caagtttcag tagtctcatg ccatgtttaa ccgaatcatg gggtgacttg 120

ccgttaaaag ttaacgattc cgaagatatg gtaatttatg ggtttctaca agacgctttt 180

agtatcggat ggacgccgtc aaatttaacg tccgaggaag tgaaactcga gccgagggag 240

gagattgagc cagctatgag tacttctgtt tctccgccga cagtggctcc agcggctttg 300

cagcctaaag gaaggcatta caggggcgtt agacaaaggc catggggaaa atttgcagcg 360

gaaataagag atccggctaa aaacggcgca cgggtttggc ttggaactta cgagtcggct 420

gaggaagccg cactcgctta tgataaagcc gcttttagga tgcgcggtac taaggctcta 480

ttgaatttcc cgcatagaat tggtttaaat gagccggagc cggttagagt gacggttaag 540

agacgattat ctgaatcggc tagttcatcg gtatcatcag cttcggaaag tggctcgcct 600

aagaggagga gaaagggtgt agcggctaag caagccgaat tagaagttga gagccgggga 660

ccaaatgtta tgaaagttgg ttgccaaatg gaacaatttc cagttggcga gcagctattg 720

gttagttaaa atatggagct aa 742

<210> 46
<211> 606
<212> DNA
<213> solanum lycopersicom

<400> 46
atgacgaaac aagatgaagg attaacatta gaactcatac gacaacatct cctcgaagat 60

ttcacaacta cagaatcatt catcgacagt ctcaattctt gtttttccga tcacatctcc 120

tcctccgatg acatctcccc tgttttcact tcagtaaaaa cagagccatc tacatccaat 180

tccctctcag attcacccaa ttcctcatac ccaaatgaac ccaactcccc aatttcccgt 240

tacttcaatc tccgctccga tttccctgaa ttcaaaatcg attcagatac catcctcagt 300

ccagttttcg acagctccgc cggttctaat gaagacaata ataagaagaa gaattacaga 360

ggggtaagga gaaggccatg ggggaaattt gcggcggaga taagagatcc aagtcgaaaa 420

ggatcgagga tttggttggg tacttttgat actgatattg atgctgctag agcttatgat 480

tgtgcagcgt ttaagatgag aggaagaaaa gctattctga attttccgtt ggatgccgga 540

aaatctggtg ctccggcgaa tgttggccgg aaaaggagaa gagagaacaa gatggagttg 600

gtgtag 606

<210> 47
<211> 783
<212> DNA
<213> solanum lycopersicom

<400> 47
atgtgtggtg gtgcaattct tgctgatatc attcctcctc gtgaccgccg tttgtcatcc 60

accgacctat ggccgactga tttctggcca atttccaccc aaaatgttcc tctcaacccc 120

aaacgagctc gaccctctac aggtggtgag cagatgaaga agaggcaaag gaagaatctt 180

tacagaggga taagacaacg tccatggggt aaatgggctg ctgaaattcg tgacccgaga 240

aaaggggtta gggtttggtt aggtactttc aacactgctg aagaagctgc aagagcttat 300

gatagagaag ctcgtaaaat caggggtaag aaagctaaag ttaatttccc caatgaagat 360

gacgaccatt actgctacag tcatccagag ccccctccct tgaacattgc ttgtgatact 420

actgttactt acaatcaaga atcaaataac tgttacccct tttactcaat cgagaacgtt 480

gaacctgtta tggaatttgc aagttataat ggaattgaag atggaggaga ggagatggtg 540

aaaaatttga ataacagggt tgtagaggaa gaggagaaaa cagaggatga agtgcagata 600

ctttctgatg agctgatggc ttatgagtca ttgatgaagt tctatgaaat accgtatgtt 660

gacgggcaat cagtggcggc gacggtgaat ccagcggcgg agaccgccgt gggcggtggc 720

tcgatggagc tttggagttt tgatgatgtt agtcgtctac aaccaagtta taatgtagtt 780

taa 783

<210> 48
<211> 669
<212> DNA
<213> solanum lycopersicom

<400> 48
atggcgccta aggaaaaaat tggtgcagtt acagctatgg caatggtgaa tttaaatgga 60

atttcgaaag aggtgcatta tagaggtgta aggaagaggc catgggggag atacgcggcg 120

gagattagag atcctgggaa aaaaagtagg gtttggttag gtactttcga tactgcggag 180

gaggcggcta gagcttatga taacgctgct agagaatttc gtggagcgaa agcgaaaact 240

aattttccga aattagaaat ggaaaaagag gaagatctga aattcgctgt gaaaaatgaa 300

atcaatcgga gtccgagtca gactagtact gtggagtcat cgagtccggt tatggttgat 360

tcatcatcgc cgttagatct aagtctctgt ggatcaatcg gcgggtttaa tcatcatacg 420

gttaagttcc cgagctccgg tggaggtttt accggttcgg tacaggcggt gaatcatatg 480

tactatatag aagcacttgc acgcgccgga gttataaagt tagaaacaaa tcggaagaaa 540

acggtagatt acctcggtgg tggtgactct gattcatcaa cggtaattga ttttatgcgt 600

gttgacgtga aatcaaccac cgccggttta aatctggatc tcaactttcc tccaccggaa 660

aacatgtga 669

<210> 49
<211> 705
<212> DNA
<213> solanum lycopersicom

<400> 49
atggatcaac agttaccacc gacgaacttc ccggtagatt ttccggtgta tcgccggaat 60

tcaagcttca gtcgtctaat tccctgttta actgaaaaat ggggagattt accactaaaa 120

gtcgacgatt ccgaagatat ggtaatttac ggtctattaa aagacgctct aagcgtcgga 180

tggtcgccgt ttaatttcac cgccggcgaa gtaaaatcgg agccgagaga agaaattgaa 240

tcgtcgcctg aattttcacc ttctccggcg gagaccacgg cagctccggc ggctgaaaca 300

ccgaaaggaa gacattatag aggcgttaga cagcgtccgt gggggaaatt tgcggcggag 360

attagagatc cggcgaagaa cggagctagg gtttggcttg gaacgtacga aacagctgaa 420

gaagctgcaa ttgcttatga taaagctgct tatagaatga gaggatcaaa agcacatttg 480

aatttcccgc accggatcgg tttgaatgaa ccggaaccgg ttcgagttac ggcgaaaagg 540

cgagcatcgc cggaaccggc aagctcgtcg ggaaacggtt ccatgaaacg gagaagaaaa 600

gccgttcaga aatgtgatgg agaaatggcg agtagatcaa gtgtcatgca agttggatgt 660

caaattgaac aattgacagg tgtccatcaa ctattggtca tttaa 705

<210> 50
<211> 675
<212> DNA
<213> solanum lycopersicom

<400> 50
atgtcaagcc cactagagat agatacttca ttttcacatt ccaatttgtt gtttttggaa 60

gatgaatcat catggagtaa tactcatgat ccatttgttg atattgatga atatctacca 120

ataattatac catgtaatga tgaagaaata gtagtagaat cctcaaacac tagtactaca 180

acaacaacaa caacaacatc aaaagtagca agtatccaaa atattcatca tgatcaagaa 240

gaggtaacat ccatagagaa aaaacatgaa gatgatcaag aaaaacatta tattggagtt 300

agaaagaggc catggggtaa atatgcatca gaaattaggg attcaacgcg taatggaatt 360

agggtttggt taggaacatt tgatactgct gaagaagctg ctttagctta tgatcaagcc 420

gcattatcaa tgaggggtcc ttggtctcta ctcaattttc caatggaaca tgttaaaaaa 480

tctcttgaaa atattgagta ttcttgtaaa gatggattat ctccagctgc tgttttaaaa 540

gctactcata aaactagaag agtcaagcac aagagaagta gtagaaagaa gaagaatgag 600

aatttggaaa atgtttttgt ttttcaagac ttgggagttg aattattaga agagctttta 660

atgacttcat catag 675

<210> 51
<211> 723
<212> DNA
<213> solanum lycopersicom

<400> 51
atggattctt cttcttcttc atctcaattc ttctactcaa tgaattctga tttaaattca 60

tcagattctt catacgaatg gtccaatttc aacacacaat cttatctccc tttcaacgtg 120

aacgactccg aagagatgct tctcttcgga gttcttaacg ctgctcatga agaaacaaca 180

tccgaaacag tcacatcgca tcgcgttaaa gaagaagaag ttacctcaga atccgaggtt 240

attgaagcaa taccggcgaa ggagaagtcg taccgaggtg ttaggaggcg tccatggggt 300

aaattcgcag cggagataag agattctacg agaaatgggg ttcgagtatg gttagggaca 360

tttgatagcg cggaagatgc tgctttagct tatgatcaag ctgcgttttc aatgaggggt 420

aattctgcca ttttgaattt tccagtggag accgttaggg attcgctacg tgacatgaaa 480

tgtcacgtag acgatgattg ctcccctgta gtggcgctta aaaagcgcca ctccatgagg 540

aaaaggagca cgaattccaa aaaagttaat agtattagta aagtagtgag ggaagttaaa 600

atggaaaatg taaataatgt agttgtgttt gaagatttgg gtgctgatta tttagaacaa 660

cttttaagta gtagttcaag tgatcaaagt agttgtgatg caacttattt tagtccatgg 720

taa 723

<210> 52
<211> 486
<212> DNA
<213> solanum lycopersicom

<400> 52
atggttccaa ctcctcaaag tgatttacct cttaatgaga atgactcaca agagatggta 60

ttatatgaag ttcttaatga agctaatgct ctaaatattc cttatttacc ccaacgaaat 120

caattactcc ctagaaataa tattcttcgt ccattacagt gcataggcaa gaaatacaga 180

ggagtacgac gtcgtccgtg ggggaaatac gctgcggaaa ttcgcgattc ggctagacat 240

ggtgcgagag tatggctagg tacgttcgaa actgctgaag aagctgcgtt agcttatgat 300

agagcggctt ttagaatgcg aggtgctaag gcactactta attttccatc tgaaatagtg 360

aacgcctctg tttcagtaga caaattaagt ttgtgctcaa atagttacac tacgaataat 420

aattcagatt caagtttaaa tgaagtttca agtggaacta atgatgtatt tgaatcaaga 480

tgttaa 486

<210> 53
<211> 1119
<212> DNA
<213> solanum lycopersicom

<400> 53
atgtgtggtg gtgcaattat ctccgatttg gtacctccta gccggatttc tcgccggtta 60

accgctgatt ttctatgggg tacatccgat ctgaacaaga agaagaagaa ccctagtaat 120

taccactcaa agcccttgag gtctaagttt attgaccttg aagatgaatt tgaagctgac 180

tttcagcact tcaaggataa ttctgatgat gatgatgatg tgaaggcatt tggccccaaa 240

tccgtgagat ctggtgattc aaactgcgaa gctgacagat cctccaagag aaagaggaag 300

aatcagtacc gggggatcag acagcgtcct tggggtaagt gggcagctga aatacgtgat 360

ccaaggaaag gtattcgagt ctggcttggt actttcaatt cagccgaaga ggcagccaga 420

gcttatgatg ctgaggcgcg aaggatcaga ggcaagaaag ctaaggtgaa ctttcctgat 480

gaagctccag tgtctgtttc aagacgtgct attaagcaaa atccccaaaa ggcacttcgt 540

gaggaaaccc tgaacacagt tcagcccaac atgacttata ttagtaactt ggatggtgga 600

tctgatgatt cgttcagttt tttcgaagag aaaccagcaa ccaagcagta cggcttcgag 660

aatgtgtctt ttactgctgt agatatggga ctgggctcag tttccccttc agctggtaca 720

aatgtttact tcagctctga tgaagcaagt aacacttttg actgctctga tttcggttgg 780

gctgaaccgt gtgcaaggac tccagagatc tcatctgttc tgtcggaagt tctggaaacc 840

aatgagactc attttgatga tgattccaga ccagagaaaa aactgaagtc ctgttccagc 900

acttcattga cagttgacgg taacactgtg aacacgctat ctgaagagct atcggctttt 960

gaatcccaga tgaagttctt gcagatccca tatctcgagg gaaattggga tgcatcggtt 1020

gatgccttcc tcaatacaag tgcaattcag gatggtggaa acgccatgga cctttggtcc 1080

ttcgatgatg taccttcttt aatgggaggt gcctactaa 1119

<210> 54
<211> 984
<212> DNA
<213> solanum lycopersicom

<400> 54
   atgtgtggtg gttctataat ctccgattac atagacccta gccggacttc tcgccggctc 60
   accgccgagt ttctatgggg tcgtttcgat ctcggtaaga agcaaaaaaa tcccaacaat 120
   tatcactcta aagctaagca tttgcgatct gaagttgttg acgactttga agccgatttt 180
   caggacttca aagagttatc cgatgatgag gatgttcaag tcgatgtcaa gccatttgcc 240
   ttctctgctt ccaaacactc tactggttcc aaatctttga aaactgttga ttcagacaag 300
   gatgctgctg ctgataaatc ctctaagaga aagaggaaga atcaatatag agggatcaga 360
   cagagacctt ggggtaagtg ggcagctgaa atacgtgacc caaggaaagg ggttcgggtc 420
   tggctgggaa ccttcaatac tgcagaagaa gctgccaaag cttatgatat tgaggcgagg 480
   aggatcagag gcaagaaggc taaggtaaac tttcctgatg aagctcccgc ccctgcatca 540
   agacacactg ttaaggtgaa tcctcagaag gtccttcctg aggagagcct gtattcactt 600
   cagtccgact cagcaatcat gaacagcgtg gaggatgacc attatgattc ttttggattt 660
   tttgaagaga aacccatgac aaaacagtat ggatatgaga atgggagcag tgcttctgca 720
   gatacgggat ttggttcgtt cgtcccttca gctggcggtg atatctactt caactctgat 780
   gtaggaagca actcttttga atgctctgat tttggttggg gagagccatg ctccaggact 840
   ccagagatat catctgttct gtcagctgct attgaatgta atgaagctca atttgttgaa 900
   gatgccaatt ctcagaaaaa gttgaaatca tgcaccaaca accccgtagc tgatgatgga 960
   aacccccgtt actatggtac ctga 984

<210> 55
<211> 747
<212> DNA
<213> solanum lycopersicom

<400> 55
atgacggaaa attcagttcc ggtgattaaa ttcactcaac acatagtaac tacaaacaag 60

catgtttttt ctgagcataa cgaaaaatcc aattcagagt tacaaagagt tgtgaggatt 120

atacttacag atgccgatgc tacagattct tccgatgatg aaggccggaa tactgtacgg 180

agagtgaaga ggcacgtgac ggagatcaac cttatgccgt caaccaaatc gatcggcgac 240

agaaaacgaa gatcggtgtc tccggattct gacgtcactc gtcggaaaaa gtttagaggc 300

gttcgtcaaa gaccgtgggg tcgttgggct gcagagattc gggacccgac cgggggaaaa 360

cgggtgtggt tgggtactta tgacacccca gaagaagcag ctgtcgttta cgataaagct 420

gcagttaagc tcaaaggtcc tgacgccgtt accaattttc cggtatcaac aacggcggag 480

gtaacggtga cggttacgga aaccgaaacc gagtctgttg ccgacggtgg agataaaagc 540

gaaaacgatg tcgctttgtc acccacctca gttctctgtg acaatgattt tgcgccgttt 600

gacaatctag ggttctgcga agtggatgct tttggtttcg acgttgattc acttttccgg 660

ctgccggatt ttgctatgac ggagaaatac tacggcgatg aattcggcga atttgacttt 720

gacgattttg cccttgaagc tcgatag 747

<210> 56
<211> 615
<212> DNA
<213> solanum lycopersicom

<400> 56
atggctaggg cacaacaaag atatcgagga gttcgacaga gacattgggg ttcttgggtc 60

tccgaaattc gccatccatt gttgaagaca agaatttggt taggcacttt tgagacagca 120

gaagatgcag caagagcata tgatgaagca gcaaggctaa tgtgtggtcc aagagctaga 180

actaatttcc catacaaccc aaacatgcca caaacatctt cctctaagct actctcaact 240

acattaacag ccaagttaca caaatgctac atggcttcac ttcaaatgac caaaacctca 300

ccacaaggac aaaaattagc aaaaaatgca accaatgttc aagaaagtgt tattaattcc 360

tataaaatga aacaacaaat gttggtacca aagccatcag tactattgac tcatcatgat 420

catcatgagg aagctaaagt agtcaacttg ggagtgggag taattaggaa agttgaagat 480

caagtacttg agggtatacc acaatttgtc aagccacttg aagatgatca cattgaacaa 540

atgattgaag aattgttgga ttatggatcc attgagcttt gctctaatgt tgttccttct 600

caccaaatcc agtga 615